# EUROPEAN PATENT APPLICATION

(11) **EP 4 024 048 A1**
(43) Date of publication of application: **06.07.2022**
(21) Application number: 20856044.1
(22) Date of filing: 28.08.2020
(51) Int. Cl.: G01N 33/554, G01N 33/68, A61K 35/28, A61K 38/17, A61P 9/10, C07K 7/08

(54) **METHOD FOR DIAGNOSING AND TREATING ATHEROSCLEROSIS BY USING NANOVESICLE TARGETING SITE OF CHANGE IN BLOOD FLOW**

(30) Priority: 30.08.2019 KR 20190107218
(71) Applicant: Numais Co., Ltd., Seoul 04799 (KR)
(72) Inventor: SUNG, Hak Joon, Seoul 06092 (KR); YOON, Jeong Kee, Seoul 08833 (KR)
(74) Representative: Miller, David James
(86) International application number: PCT/KR2020/011581
(87) International publication number: WO 2021/040473

(57) **Abstract**

The present invention relates to a method for diagnosing and treating atherosclerosis by using nanovesicle targeting a site of change in blood flow, and more specifically, the present invention provides a stem cell-derived nanovesicle, which is an anti-atherosclerosis diagnosis and treatment platform using a peptide capable of targeting a disturbed blood flow, wherein the nanovesicle provides strong anti-inflammatory and pre-endothelial recovery effects similar to that of a mesenchymal stem cell, and thus it can be used as a new diagnosis and treatment agent capable of preventing the onset of atherosclerosis.

## Description

### [Technical Field]

The present invention relates to a method of diagnosing and treating atherosclerosis using nanovesicles targeting a site with a change in blood flow.

### [Background Art]

Atherosclerosis is a leading cause of death, but current diagnosis methods cannot yet detect early etiological signals of this disease associated with an irreversible cascade. Although there remains a substantial risk of disease progression, conventional preventive and therapeutic options targeting a low cholesterol level, blood pressure or plaque formation have been widely used. The occurrence of disturbed blood flow, which is an early atherosclerotic event arising at the branch points, curved regions or distal to stenosis, results in dysfunction of endothelial cells (ECs). Under normal blood flow, ECs are aligned to a blood flow direction, and maintain anti-inflammatory and anti-thrombotic functions. In contrast, in the case of atherosclerosis, disturbed blood flow activates EC dysfunction as well as increased inflammation and thrombotic events, ultimately causing atherosclerosis. Disturbed blood flow-targeting theragnostics that can present a promising solution for this fatal chronic disease must be thoroughly demonstrated.

It has been reported that mesenchymal stem cells (MSCs) maintain therapeutic promise for anti-atherosclerosis treatments by regulating the immune response and attenuating the proliferation of vascular smooth muscle cells (VSMCs). However, their low survival rate and insufficient target efficiency must be overcome for clinical application. MSC-derived nanovesicles have emerged as therapeutic nanocarriers with prolonged circulation time in the body, which may not only have cell-originating anti-inflammatory and pre-regenerative characteristics but also promote cellular interactions. Despite attempts to develop nanovesicle-based therapeutics for several fatal diseases and injuries including a cardiovascular disease, renal injury, a liver disease and a neurological disease, maintaining size uniformity and the consistency of an active ingredient from a high yield in a long and difficult production process is still a challenge. A recent alternative engineering approach can produce cell-derived nanovesicles in 100-fold or higher yield by physically disrupting cells through micropore filtration and inducing self-assembly of fragments of the resulting cell membrane and internal contents.

An irreversible cascade leads to the critical pathogenesis of atherosclerosis, which presents an unmet need for early diagnosis and prevention. Disturbed blood flow formation is one of the fastest atherosclerotic events that increases endothelial permeability and subsequent monocyte recruitment.

### [Disclosure]

### [Technical Problem]

The present invention is directed to providing stem cell-derived nanovesicles displaying a peptide capable of targeting a disturbed blood flow site causing atherosclerosis on their surfaces and a method of producing the same.

The present invention is also directed to providing a use of the nanovesicles for preventing, diagnosing, and treating atherosclerosis.

However, technical problems to be solved in the present invention are not limited to the above-described problems, and other problems which are not described herein will be fully understood by those of ordinary skill in the art from the following description.

### [Technical Solution]

To achieve the above-described purposes, the present invention provides stem cell-derived nanovesicles displaying a peptide capable of targeting a disturbed blood flow site causing atherosclerosis on their surfaces.

The present invention also provides a method of producing the nanovesicles, which includes obtaining nanovesicles displaying a peptide targeting disturbed blood flow sites on their surfaces from stem cells transfected with a vector into which coding sequences of a signal peptide, a disturbed blood flow site-targeting peptide and a transmembrane protein are sequentially inserted.

The present invention also provides a composition for diagnosing atherosclerosis, which includes the nanovesicles.

The present invention also provides a composition for preventing or treating atherosclerosis, which includes the nanovesicles.

The present invention also provides a method of treating atherosclerosis, which includes administering a therapeutically effective amount of the nanovesicles into a subj ect.

### [Advantageous Effects]

The present invention provides stem cell-derived nanovesicles, which are an anti-atherosclerosis theragnostic platform using a peptide capable of targeting a disturbed blood flow site, and the nanovesicles can be used as a novel theragnostic agent which can prevent the onset of atherosclerosis by providing potent anti-inflammatory and pre-endothelial repair effects similar to those of MSCs.

### [Description of Drawings]

FIG. 1A is the operation mechanism of PREY/MSC-derived nanovesicles (PMSC-NVs) and a schematic diagram illustrating PREY peptide display, and specifically, a disturbed blood flow site-targeting peptide (PREY) is selected from phage display screening and displayed on the outside of an MSC membrane (PMSC) through transfection and expression of a specifically designed plasmid.
FIG. 1B shows that PMSC-derived NVs (PMSC-NVs) are produced by continuous extrusion process using a series of membranes controlled to a micropore size, and they include transmembrane and intracellular components, which are the same as those of PMSC's.
FIG. 1C shows that a partial carotid ligation (PCL) model is used in an *in vivo* test, in which PMSC-NVs are administered intravenously and circulate throughout the body to target a disturbed blood flow, thereby testing the theragnostic performance thereof.
FIG. 2A shows a schematic diagram of the design of a plasmid for expressing PREY on the outer layer of the MSC membrane. Green fluorescence protein (GFP: internal membrane signal)-transmembrane protein-v5 tag (outer membrane signal)-PREY was designed to validate the expression and location of PREY.
FIG. 2B is the results of quantitatively comparing transfection efficiency through FACS (x-axis: GFP+ cells/ y-axis: V5 tag+ cells) in two types of hMSC types (ASCs and BMSCs) paired with three types of transmembrane proteins (CD86, CD105 and CD271). Here, the CD271-ASC pair was previously selected due to having the highest transfection efficiency.
FIG. 2C is the results of evaluating PREY-CD271 plasmid dose-dependent apoptosis (0X: electroporation without plasmid and IX, 2X: 100, 200 ng plasmid/10⁵ cells) using Alexa Fluor 488-conjugated Annexin V, measured 30 minutes after transfection.
FIG. 2D shows the locations of GFP (green internal membrane signal) and PREY-v5 tag (red external membrane signal), visualized in PMSCs after transfection of PREY-CD271-v5 tag plasmids into ASCs, and the red and green signals are mainly observed outside and inside the cell membrane, respectively.
FIG. 2E is the results of measuring the size and the morphology distribution of PMSC-NVs using transmission electron microscopy (TEM), and a yellow dotted line represents the PMSC-NV membrane.
FIG. 2F is the results of analyzing the expression of transfected genes and the cell membrane protein CD9 through western blotting.
FIG. 2G is the results of analyzing the preservation of internal miRNA components using a miRNA array.
FIG. 3 is a schematic diagram of a vector design for expressing a PREY fusion protein, and a sequence gene of the PREY fusion protein is inserted into a common CMV promoter backbone along with an ampicillin resistance gene for cloning.
FIG. 4A shows fluorescent images (blue: DAPI-stained nucleus) representing the transfection efficiency of IX plasmids in BMSCs and ASCs, respectively, 24 hours after transfection of PREY-CD271 plasmids to determine the transfection efficiency according to hMSC type.
FIG. 4B is the results of measuring the number of viable cells depending on the transfection dose (0X, 0.5X, IX or 2X) of PREY-CD271 plasmids in BMSCs and ASCs, in which due to the higher number of viable cells in ASCs, they were selected as a hMSC type (^{∗}p < 0.05 BMSC/ASC, #p < 0.05/ 0X BMSC, $p < 0.05/ 0X ASC).
FIG. 4C is the results of analyzing transfection efficiency depending on the PREY-CD271 plasmid dose (0.5X, IX or 2X) in ASC through FACS.
FIG. 5 is the confocal imaging results to show the expression of PREY-CD271, in which PREY-CD271 expression can be confirmed by the expression of GFP and PREY-V5 tag on the cell membrane of adjacent ASCs (blue: DAPI-stained nucleus).
FIG. 6 is the western blotting results to analyze the expression of transfected genes and the level of the cell membrane protein CD9 in samples before and after extrusion of NVs from ASCs (PMSC-NVs) after transfection (MSC-NV: NV extrusion from ASCs which are not transfected, ^{∗}p < 0.05).
FIG. 7A illustrates the anti-inflammatory effects caused by the monocytes activated by BMSC (BMSC-NV)- or ASC (ASC-NV)-derived MSC-NV (without PREY transfection) treatments, in which the monocytes absorb MSC-MVs, thereby exhibiting anti-inflammatory and phagocytosis-inhibitory effects.
FIG. 7B is the imaging results (blue: DAPI-stained nucleus) visualizing RAW264.7 cells and MSC-NVs using DiO (green) and DiI (red), respectively, indicating that two MSC-NV types are efficiently internalized into macrophages.
FIG. 7C is the qRT-PCR results of measuring the mRNA expression levels of anti-inflammatory markers (IL-10 and IL-13) and pro-inflammatory markers (IL-1β and TNF-α) when murine Raw 264.7 cells are treated with LPS.
FIG. 7D is the dot blot assay results of using a conditioned medium, showing that the amount of black dots in the lower right of each type of anti-inflammatory cytokine is decreased in the group treated with nanovesicles. The upper left black dots (displayed with a solid circle) show that the amount of anti-inflammatory cytokines in a test medium is similar in all groups.
FIG. 7E is the results of analyzing phagocytotic activity by measuring the amount of internalized E. coli particles (green), showing that there was no significant difference between all MSC-NV types, and the Oil Red O staining result (lower panel) showing a decrease in ox-LDL uptake in all NV-treated groups except a BMSC-NV group, and the lower graphs show quantitative results.
FIG. 8A is a diagram showing vascular protective effects of ECs due to the treatments with BMSC (BMSC-NV)- or ASC (ASC-NV)-derived MSC-NVs (without PREY transfection), indicating that, as MSC-NVs provide EC protection and inhibit monocyte recruitment, EC dysfunction is repaired by MSC-NV uptake.
FIG. 8B is the imaging results visualizing immortalized mouse ECs (iMAECs) and MSC-NVs using DiO (green) and DiI (red), respectively, showing that both MSC-NV types are effectively internalized into the iMAECs (blue: DAPI-stained nucleus).
FIG. 8C is the qRT-PCR results of measuring gene expression levels of EC dysfunction markers (E-selectin, ICAM-1 and VCAM-1), showing that the expression of each gene is reduced by all MSC-NV treatments.
FIG. 8D is the immunostaining image to anlalyze the protein expression of VCAM-1 (green) in iMAECs and its quantification result (blue: DAPI-stained nucleus).
FIG. 8E is the results to show the vascular protective effects of BMSC-NV or ASC-NV treatments on the disruption of EC angiogenesis induced by cyclosporin A (CyA), showing that there is no significant difference between the two MSC-NV types (^{∗}p < 0.05/ LPS/saline-treated group).
FIG. 9 is the result of analyzing a pro-angiogenic effects of MSC-NVs in response to the disturbance of EC angiogenesis by CyA treatments, and it shows images of HUVECs stained with Calcein AM and the quantitative analysis result of vasculature factors (saline-treated group/ ^{∗}p < 0.05).
FIG. 10 is the results to show the pro-EC repair effects of PMSC-NVs in response to VCAM1 expression, angiorrhexis, and anti-angiogenesis.
FIG. 11 shows the images of a murine PCL model before and after ligation (left/right), in which three of four left common carotid artery (LCA) branches (external carotid artery (ECA), internal carotid artery (ICA), and occipital artery (OA)) are ligated using 10-0 nylon suture, and the superior thyroid artery (STA) is not ligated.
FIG. 12 shows the results of verifying disturbed blood flow formation after ligation in the LCA by Doppler ultrasound imaging in murine PCL models, in which RCA images (top) show normal pulsating laminar flows, whereas LCA images (bottom), compared with the RCA images, show abnormal blood flows moving forward and backward with a significant decrease in liquid flow.
FIG. 13A shows the theragnostic effects of PMSC-NVs on disturbed blood flow sites in a murine PCL model. Vivotrack680-labeled MSC-NVs and PMSC-NVs were intravenously administered into mice for systemic circulation three days after PCL surgery, and the *in vivo* distribution of MSC-NVs, PMSC-NVs, and PMSCs in the entire mouse body was analyzed using an *in vivo* imaging system 24 hours after surgery.
FIG. 13B shows the results of confirming MSC-NV, PMSC-NV, and PMSC distributions using an *in vivo* imaging system (IVIS) in RCAs (control) and LCAs (ligated) harvested after an experiment was performed in the same manner as described in FIG. 13A (^{∗}p < 0.05 between groups).
FIG. 13C shows the expression levels of the filamin A protein and the co-existence of MSC-NVs and PMSC-NVs in harvested RCAs and LCAs by immunostaining and their quantitative result (^{∗}p < 0.05 and ^{∗}p < 0.001 between groups).
FIG. 13D is the images of LCAs stained with H&E on day 14 after PCL (top row), which show the suppression of angiogenesis by PMSC-NV treatments through the quantitative analysis of neointimal structure parameters, and macrophage recruitment by ECs through immunostaining of CD68 (middle row) and VCAM-1 (bottom row) in collected LCAs (blue: DAPI-stained nucleus). Here, white lines indicate the inner boundary of the media layer facing the intima (^{∗}p < 0.05/ saline-treated group).
FIG. 14 shows the images to analyze the *in vivo* distributions of MSC-NVs, PMSC-NVs, and PMSCs in major organs after injection into PCL mice through imaging (left) of an IVIS system and their quantitative analysis results (HT: heart, LG: lung, LV: liver, SP: spleen and KN: kidney). Here, the lowest fluorescent intensity of PMSC-NVs in LG among the test groups indicates the synergistic role of PREY and NV that enables the cells to avoid lung capillary entrapment (right) (^{∗}p < 0.001 between groups).
FIG. 15 is the images to show the accelerated atheroma formation in LCAs of ApoE KO and normal mice (balb/c) fed an atherosclerotic diet on day 14 after ligation and its quantitative analysis result. Even with an atherosclerotic diet, unligated mice exhibit no visible atheroma formation in the LCA (ND: non-detectable).
FIG. 16 shows the mouse organs (lung, liver, kidney, and spleen) stained with H&E on day 11 after a PCL mouse model is treated with MSC-NVs or PMSC-NVs.
FIG. 17A shows an image of a porcine PCL model formed by inducing the surgical ligation of the LCA using a stainless-steel rod (diameter=0.9 mm), followed by removal of the rod after ligation, and sonographic images showing a normal condition (top) and partial occlusion of the LCA (bottom) after ligation surgery (yellow arrow: upper and lower parts of ligation).
FIG. 17B is the Doppler ultrasound imaging result to show the formation of disturbed blood flow in the distal region of the ligation point of the LCA group in contrast to the unidirectional laminar flow in normal and RCA groups (yellow arrow: blood flow direction).
FIG. 17C is the images to show the filamin A expression of endothelial layers in the RCA and LCA.
FIGS. 17D to 17G are the IVIS and immunostaining results of analyzing blood vessels collected 24 hours after NVs are intravenously administered on day 3 after porcine PCL surgery, showing effective PMSC-NV targeting of disturbed blood flow sites in the porcine model in the collected LCA (FIGS. 17D and 17E) and aortic arch (FIGS. 17F and 17G) (region of natural disturbed blood flow formation) (^{∗∗∗}p < 0.001 between groups).
FIG. 18 shows the IVIS images of the RCA and LCA (induced disturbed blood flow; left) and aortic arch (natural disturbed blood flow; right) 24 hours after PMSC-NV treatment in a porcine PCL model.
FIG. 19A illustrates a process in which human coronary artery endothelial cells (hCAEC) are cultured under unidirectional laminal flow or disturbed blood flow for one day, 12 hours after adherent culture, and treated with NVs for 1 hour in order to analyze the PMSC-NV targeting efficiency of arterial ECs under disturbed blood flow using a microfluidic model.
FIG. 19B shows normal (laminar) flow and disturbed blood flow pattern plots.
FIG. 19C the immunostaining results to show the alignment of hCAECs by filamin A expression (green) as well as F-actin expression (red).
FIG. 19D is the quantitative results to analyze the F-actin alignment in a flow direction based on the results of FIG. 19C.
FIG. 19E is the quantitative results to analyze the expression level of filamin A in the cytoplasm under disturbed blood flow based on the results of FIG. 19C (^{∗}p < 0.05 between groups).
FIG. 19F is the quantitative results to analyze the *in vitro* targeting efficiency of MSC-NVs and PMSC-NVs for hCAECs at disturbed blood flow sites by measuring NV fluorescent intensity based on the results of FIG. 19C (^{∗∗∗}p < 0.001 between groups).
FIG. 19G is the results of confirming PREY targeting of filamin A by quantitatively analyzing the co-existence of NVs and filamin A based on the results of FIG. 19C (^{∗∗∗}p < 0.001 between groups).
FIG. 20 is the IVIS imaging results to show the efficiency of PMSC-NVs targeting human aortic ECs (hAECs) using an *in vitro* microfluidic model.

### [Modes of the Invention]

Hereinafter, the configuration of the present invention will be described in detail.

The present invention relates to stem cell-derived nanovesicles displaying a disturbed blood flow site-targeting peptide on their surface.

In addition, the present invention provides a method of preparing the nanovesicles, which includes obtaining nanovesicles displaying a disturbed blood flow site-targeting peptide on their surface from stem cells transfected with a vector into which coding sequences of signal peptide-disturbed blood flow site-targeting peptide-transmembrane protein-green fluorescent protein (GFP) are sequentially inserted.

The "disturbed blood flow" used herein refers to abnormal and irregular blood flow due to the structural characteristics of a blood vessel, and it is an event of early atherosclerosis causing vascular endothelial cell dysfunction.

The "nanovesicles (NVs)" used herein are prepared through disruption of cells by artificially passing through a filter and formed by their self-assembly mechanism, and they are understood as being different from exosomes extracted from cells.

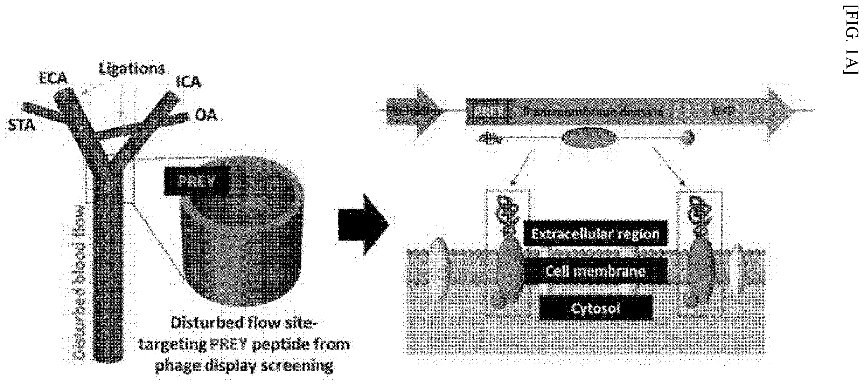

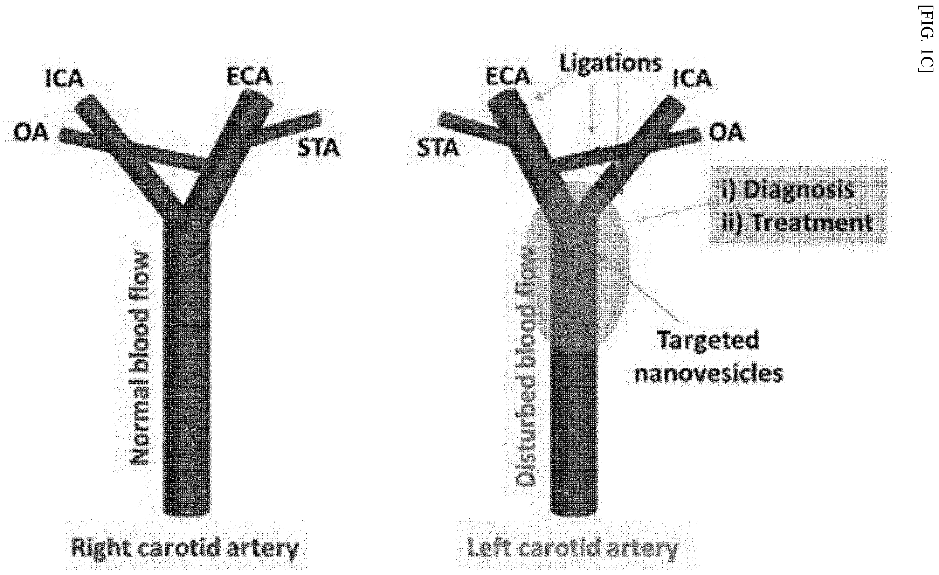

The present invention provides stem cell-derived nanovesicles functionalized with a peptide targeting a disturbed blood flow site (see FIGS. 1A to 1C).

The disturbed blood flow site-targeting peptide may be selected from the group consisting of SEQ ID NOs: 1 to 5. More specifically, SEQ ID NO: 1 is a PREY peptide having an amino acid sequence of GSPREYTSYMPH, SEQ ID NO: 2 is a myoferlin peptide having an amino acid sequence of SPREYTSYMPH, SEQ ID NO: 3 is an Eyes absent homolog 1 isoform 2 peptide having an amino acid sequence of SLSSYNGSALAS, SEQ ID NO: 4 is a partial peptide of a zinc finger protein having an amino acid sequence of ACNTGSPYEC, and SEQ ID NO: 5 is a Calsyntenin 1, isoform CRA_b peptide having an amino acid sequence of ACTPSFSKIC.

In the nanovesicles of the present invention, it is easy to confirm the expression of a disturbed blood flow site-targeting peptide, compared to the conventional disturbed blood flow site-targeting peptide-liposome, and a rate of the disturbed blood flow site-targeting peptide expression may be increased by GFP/v5tag FACS sorting in a cell stage prior to nanovesicle extraction. In addition, compared to liposomes, nanovesicles contain a stem cell-derived therapeutic substance for suppressing atherosclerosis. The nanovesicles have advantages over other chemical drugs in terms of stability or the risk of side effects. In addition, due to the use of patient-derived autologous stem cells, the nanovesicles are free from immune responses, and particularly, mesenchymal stem cell-derived nanovesicles are manufactured from cells free from host-immune rejection. Since PMSC-NVs possessing a surface marker of the mesenchymal stem cells also have the above-described characteristic, there also is a possibility for allotransplantation, and in the present invention, through a pre-clinical trial using mice and pigs, the possibility is partially confirmed. In addition, due to the host-immune rejection avoidance mechanism of mesenchymal stem cells, the mesenchymal stem cell-derive nanovesicles are expected to be free from macrophages compared to liposomes, which is expected to lead to an increase in targeting efficiency.

The nanovesicles may be separated through the size-controlled extrusion of the stem cells transfected with a vector into which coding sequences of signal peptide-disturbed blood flow site-targeting peptide-transmembrane protein (TMP)-green fluorescent protein (GFP) are sequentially inserted using a conventional transformation technology.

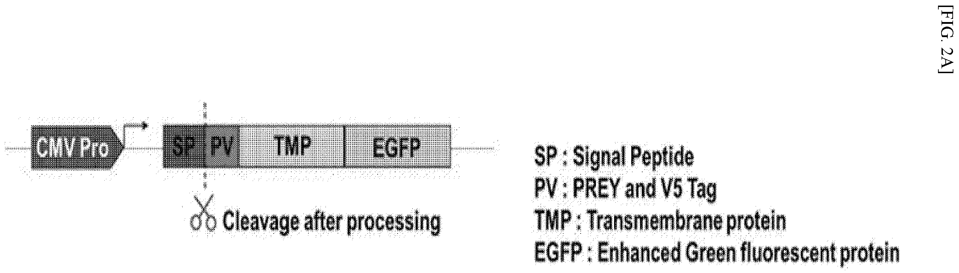

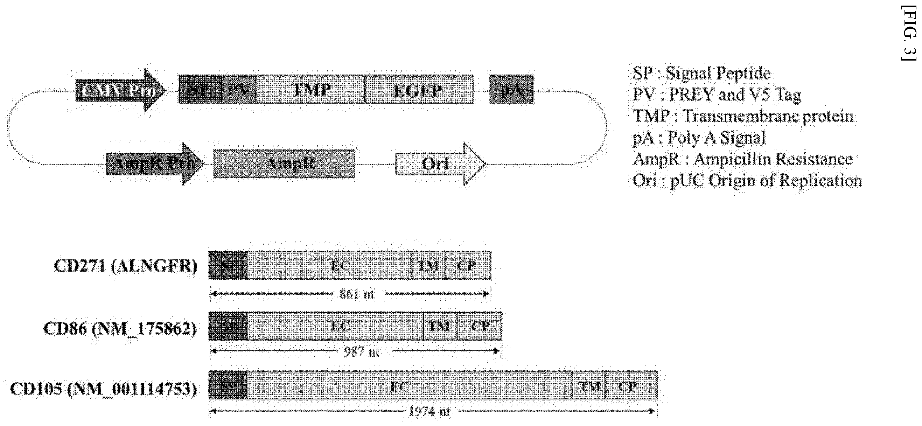

According to one embodiment of the present invention, plasmid DNA designed to functionalize a PREY peptide targeting a disturbed blood flow site to display it on the outside of an MSC membrane and produce NVs through physical cell disruption and subsequent self-assembly is used. To this end, a plasmid composed of external N-terminus-promoter-signal peptide-PREY-v5 tag-TMP-GFP-internal C-terminus structure is constructed (see FIGS. 2A to 2G, and 3). The signal peptide may induce localization of the PREY peptide to the outside of the cell membrane and deactivate an induction signal through cleavage. The v5 tag and GFP may be used to monitor the location and expression level of PREY. According to one embodiment of the present invention, GFP is expressed inside the cell membrane, and the v5 tag and the PREY peptide are expressed outside the cell membrane.

In addition, to improve the expression level of the disturbed blood flow site-targeting peptide, the ability of a peptide searching for and targeting the disturbed blood flow site may be maximized by using a transmembrane protein (TMP). Accordingly, the transmembrane protein may be i) a protein expressed in stem cells or nanovesicles. For example, as the transmembrane protein, an exosome marker such as CD86 and mesenchymal stem cell markers such as CD105 or CD271 may be used.
ii) The N-terminus and the C-terminus of the protein have to face in opposite directions with the cell membrane interposed therebetween.

Preferably, CD271 with high transfection efficiency may be used.

As the signal peptide, signal peptide F (BKU002587, Korea Human Gene Bank, Republic of Korea) and signal peptide R (BKU008396, Korea Human Gene Bank, Republic of Korea) may be used, but the present invention is not limited thereto.

The coding sequence of signal peptide-disturbed blood flow site-targeting peptide-transmembrane protein-green fluorescent protein (GFP) is a nucleic acid sequence, and the nucleic acid is used in the broadest sense, which includes single stranded (ss) DNA, double stranded (ds) DNA, cDNA, (-)-RNA, (+)-RNA, and dsRNA. Preferably, the nucleic acid is ds DNA.

Preferably, when DNA is selected as the coding sequence of signal peptide-disturbed blood flow site-targeting peptide-transmembrane protein, it may be used while being inserted into an expression vector.

The term "vector" used herein refers to a nucleic acid molecule capable of delivering another nucleic acid linked thereto. As one type of vector, "plasmid" refers to a circular double-stranded DNA loop into which an additional DNA segment may be ligated. Another type of vector is a viral vector that can ligate an additional DNA segment into a viral genome. Some vectors may be self-replicated in host cells when introduced into host cells (e.g., bacterial vectors having a bacterial replication origin and episomal mammalian vectors). Other vectors (e.g., non-episomal mammalian vectors) may be integrated into the genome of a host cell when introduced into the host cell and replicated with the host genome. In addition, some vectors may direct the expression of a gene to which they are operably linked. Such a vector used herein is called a "recombinant expression vector" (or simply, called "expression vector"). Generally, the expression vector useful for a recombinant DNA method is typically a plasmid form, and since the plasmid is the most common vector type, the "plasmid" and the "vector" may be used interchangeably. However, the present invention includes different types of expression vectors such as viral vectors providing equivalent functions (e.g., an adenovirus vector, an adeno-associated virus (AAV) vector, a herpes virus vector, a retrovirus vector, a lentivirus vector, a baculovirus vector). Preferably, a lentivirus vector can be used. Transformation includes any method to introduce a nucleic acid into an organism, a cell, a tissue or organ, and it may be performed by selecting appropriate standard techniques depending on host cells as known in the art. These methods include electroporation, protoplast fusion, calcium phosphate (CaPO₄) precipitation, calcium chloride (CaCl₂) precipitation, stirring using silicon carbide fibers, agrobacteria-mediated transformation, PEG, dextran sulfate, and Lipofectamine, but the present invention is not limited thereto.

The stem cells may be stem cells derived from one or more types of tissue selected from the group consisting of bone marrow, the umbilical cord, umbilical cord blood, the placenta, blood, skin, adipose tissue, nervous tissue, the liver, the pancreatic duct, muscle and the amniotic membrane; mesenchymal stem cells; embryonic stem cells; or induced pluripotent stem cells. Preferably, stem cells containing miRNAs such as miR-21, miR-132, miR-10, miR-146, miR-143 and let 7 having an anti-atherosclerotic characteristic may be used. For example, adipose-derived stem cells with high transfection efficiency and a low apoptosis rate may be used.

According to one embodiment of the present invention, the nanovesicles of the present invention may be obtained through extrusion while porous membranes with sizes of 10 µm, 5 µm and 400 nm are sequentially changed for stem cells transfected with a vector expressing a PREY peptide. According to one embodiment of the present invention, as a result of TEM and DLS analyses, a uniform distribution of nanovesicles having an average diameter of about 47.2±12.1 nm and about 83.7±20.6 nm, respectively, may be obtained, and the diameters are smaller than 14.9±2.0 µm of stem cells measured by DLS.

The nanovesicles of the present invention contain intracellular components even after extrusion, in comparison with intracellular components of stem cells, and the levels of anti-atherosclerotic miRNA, for example, miR-21, miR-132, miR-10, miR-146, miR-143 and let 7, are increased upon extrusion.

When macrophages activated by LPS treatments are treated with the nanovesicles of the present invention, the expression of an anti-inflammatory cytokine gene increases, exhibiting an anti-inflammatory effect. In addition, the foam cell formation of macrophages caused by the uptake of oxidized LDL results in the induction of phenotypic changes and the internal growth of VSMCs, therefore, the region in which such results are obtained is important for the development of atherosclerosis. These results show that the nanovesicles can prevent an atherosclerosis process.

In addition, as a result of testing an endothelial cell repair effect by nanovesicle treatments by activating the pro-inflammatory dysfunction of iMAECs, since endothelial cells dominantly express E-selectin, ICAM-1, and VCAM-1 to recruit inflammatory cells in dysfunction activation, all of them are upregulated by LPS treatments when gene expression of these markers are measured. When treated with the nanovesicles, however, they are significantly downregulated, and therefore, the nanovesicles exhibit an endothelial cell repair effect. Moreover, by the CyA treatments in HUVECs, the nanovesicles can improve the pre-endothelial cell recovery and pre-angiogenic effects.

In addition, as a result of testing a disturbed blood flow site-targeting effect of nanovesicles in murine and porcine PCL models, it can be seen that a PREY peptide, which is the disturbed blood flow site-targeting peptide, targets filamin A overexpressed in the disturbed blood flow site, which may confirm a synergistic theragnostic effect of preventing the early progression of atherosclerosis. Moreover, in an *in vitro* microfluidic model, the nanovesicles co-exist with filamin A, and increase under a disturbed blood flow condition, which demonstrates the theragnostic potential of the nanovesicles.

In addition, the nanovesicles of the present invention reduce the expression level of the control without noticeable toxic effects in the heart, lungs, liver and spleen after systemic circulation.

Accordingly, the present invention also provides a composition for preventing, diagnosing and treating atherosclerosis, which includes the nanovesicles.

The composition for preventing or treating atherosclerosis of the present invention may include an active ingredient and an active or inactive pharmaceutically acceptable carrier, which are used for a composition suitable for a preventive, diagnostic, or therapeutic use *in vitro, in vivo,* or *ex vivo.*

The pharmaceutically acceptable carrier includes any pharmaceutically carrier which can be mixed with nanovesicles, like protein excipients including a phosphate-buffered saline (PBS) solution, serum albumin such as human serum albumin (HSA), recombinant human albumin (rHA), gelatin and casein. Examples of carriers, stabilizers and adjuvants can be found in Martin REMINGTON'S PHARM. SCI, 18th Ed. (Mack Publ. Co., Easton (1995)) and the "PHYSICIANS DESK REFERENCE", 58nd Ed., Medical Economics, Montvale, NJ. (2004). The term "carrier" may include a buffer solution or a pH adjuster, and the typical buffer solution is a salt prepared from an organic acid or base. Representative buffer solutions include organic acid salts such as a citric acid salt, an ascorbic acid salt, a gluconic acid salt, a carbonic acid salt, a tartaric acid salt, a succinic acid salt, an acetic acid salt, and a phthalic acid salt; Tris, tromethamine hydrochloride and a phosphate buffer. Additional carriers include polymeric excipients/additives, for example, polyvinylpyrrolidone, Ficoll (polymer sugar), dextrates (e.g., cyclodextrins, such as 2-hydroxypropyl-quadrature, 2-hydroxypropyl-cyclodextrin), polyethylene glycol, antioxidants, anti-static agents, surfactants (e.g., polysorbates such as "TWEEN 20" and "TWEEN 80"), lipids (e.g., phospholipids and fatty acids), steroids (e.g., cholesterol) and chelating agents (e.g., EDTA). An anti-icing agent or freezing point depressing agent may also be included.

The composition for preventing, diagnosing, or treating atherosclerosis may be prepared in various appropriate formulations. For example, formulations and carriers suitable for administration via parenteral routes such as intra-arterial (at a joint), intravenous, intramuscular, intradermal, intraperitoneal, intranodal and subcutaneous routes, can include antioxidants, buffers, bacteriostats, solutes that allow a formulation to have the same osmotic pressure as the blood of a target recipient, and aqueous and non-aqueous sterile suspensions including a suspending agent, a solubilizing agent, a thickening agent, a stabilizing agent and a preservative. Intravenous or intraperitoneal administration are preferable methods. The dosage of cells administered to a subject is an amount effective to achieve a desired beneficial therapeutic response in the subject over time. For example, before injection, a blood sample is obtained from the subject and then stored, and then used in subsequent analysis and comparison. Generally, at least 10⁴ to 10⁶, and typically 1×10⁸ to 1×10¹⁰ cells may be intravenously or intraperitoneally injected into a 70-kg patient for approximately 60 to 120 minutes. In consideration of overall health conditions and the weight of a subject, the nanovesicles of the present invention are administered in a proportion determined by LD-50 (or other toxicity measurement methods) according to cell type and a side effect according to cell type at various concentrations. The cells may be administered at one time or in several divided portions. The nanovesicles of the present invention may supplement treatments for other specific symptoms using some known conventional therapeutic methods including a cytotoxic agent, a nucleotide analogue, or a biological response modifier. Similarly, the biological response modifier may be selectively added to the treatments with the nanovesicles of the present invention.

The present invention also provides a method of treating atherosclerosis, which includes administering a therapeutically effective amount of the nanovesicles into a subj ect.

As the nanovesicles and the administration method used in the atherosclerosis treatments are described above, in order to avoid excessive complexity of the specification, descriptions of the common contents between these are omitted.

The subject may be a mammal such as a dog, a cat, a rat, a mouse, or a human, but the present invention is not limited thereto.

Hereinafter, the advantages and features of the present invention and the methods of accomplishing the same will become apparent with reference to the detailed description of exemplary embodiments and the accompanying drawings. However, the present invention is not limited to the exemplary embodiments disclosed below, and it may be embodied in many different forms. These exemplary embodiments are merely provided to complete the disclosure of the present invention and fully convey the scope of the present invention to those of ordinary skill in the art, and the present invention should be defined by only the accompanying claims.

Hereinafter, the present invention will be described in more detail with reference to examples. The examples are merely provided to more fully describe the present invention, and it will be obvious to those of ordinary skill in the art that the scope of the present invention is not limited to the following examples.

### <Example 1> Preparation and characterization of PREY peptide-nanovesicles (NVs)

### (Plasmid design and cloning for PREY expression)

A plasmid displaying and localizing PREY on a cell membrane consists of external N-terminus-signal peptide-PREY-V5-TMP-GFP-internal C-terminus. As the signal peptide, Signal peptide F (BKU002587, Korea Human Gene Bank, Republic of Korea) or Signal peptide R (BKU008396, Korea Human Gene Bank, Republic of Korea) are used. In the plasmid sequence, i) the signal peptide induces localization of the PREY peptide to the outside of the cell membrane; and ii) the V5 tag and GFP monitor the location and the expression level of PREY. An expression vector was cloned using a NEB Gibson Assembly kit (New England Biolabs, MA) according to the manufacturer's instructions. Signal peptides and each type of transmembrane protein were amplified by PCR using the following templates: Signal peptide F (BKU002587, Korea Human Gene Bank, Republic of Korea), Signal peptide R (BKU008396, Korea Human Gene Bank, Republic of Korea), NGFR (Addgene plasmid #27489; Addgene, MA) for CD86, CD105, and cleaved CD271 (LNGFR). The vector components were inserted into a Cas9-digested p3S-Cas9-HNa (Addgene plasmid #104171) backbone with plasmid synthesis (Macrogen, Republic of Korea). According to the above procedure, PCR amplification and Gibson cloning were performed. All primers and plasmids are listed in Tables 1 and 2.

**[Table 1] List of primers used in cloning**

| **Insert** | | **Name of primer** | **Sequence (5'→3')** | **Template** | **Notes** |
|---|---|---|---|---|---|
| CMV promoter backbone | All | pCMV Backbone_F<S EQ ID NO: 6> | TAACTCGAGCATGCATCT AGAG | p3S-Cas9-HN (A gift from Jin-Soo Kim; Addgene plasmid # 104171) | linearized p3S-Cas9-HN plasmid without Cas9 Sequence |
| | | pCMV Backbone _R<S EQ ID NO: 7> | GGTGGCAAGCTTGGGTCT CC | | |
| GS linker and EGFP | All | mEGFP_F<SE Q ID NO: 8> | GAAGCGGAGGAGGAGGA AGCGGAGGAGGAGGTAG CGTGAGCAAGGGCGAG | pC015-dLwCas13a-NF (A gift from Feng Zhang; Addgene plasmid # 91905) | - |
| | | mEGFP_R<SE Q ID NO: 9> | ATAGGGCCCTCTAGATGC ATGCTCGAGTTATTACTTG TACAGCTCGTCCATG | | - |
| Signal peptide and PREY | CD86 | CD86 Signal_F<SEQ ID NO: 10> | CTCACTATAGGGAGACCC AAGCTTGCCACCATGGAT CCCCAGTGCACTAT | NM_175862 (BKU002587, Korea Human Gene Bank) | - |
| | | CD86_Signal_ PREY_R<SEQ ID NO: 11> | GTGAGGCATATAGGAGGT GTACTCGCGGGGACTGCC AGCTTGAATCTTCAGAGG AG | | |
| | CD271 | NGFR Signal_F<SEQ ID NO: 12> | CTCACTATAGGGAGACCC AAGCTTGCCACCATGGAC [?] GGGCCGCGCCT [?] | NGFR (A gift from Warren Pear; Addgene plasmid # 27489) | - |
| | | NGFR Signal R<SEQ ID NO: 13> | CATATAGGAGGTGTACTC [?] GCGGGGACTGCCGGCACC [?] TCCAAGGGACACCC | | - |
| | CD105 | CD105 Signal_F<SEQ ID NO: 14> | CTCACTATAGGGAGACCC AAGCTTGCCACCATGGAC CGCGGCACGCTCCC | NM_001114753 (BKU008396, | - |
| | | CD105 Signal R<SEQ ID NO: 15> | 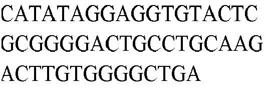 | Korea Human Gene Bank) | - |
| PREY and V5 Tag | All | V5 tag_PREY_F< SEQ ID NO: 16> | GGCAGTCCCCGCGAGTAC ACCTCCTATATGCCTCAC GGCAAGCCCATCCCCAAC CC | Primer extension without template | PREY and V5 tag |
| | CD86 | V5 tag_CD86_R< SEQ ID NO: 17> | GCATGGCAGGTCTGCAGTCTCATTGAAAT AGGTGCTGTCCAGGCCCAGCA | | V5 tag and 30bp homology with CD86 mature peptide |
| | CD271 or CD105 | V5 Full AS<SEQ ID NO: 18> | GGTGCTGTCCAGGCCCAGCAGGGGGTTG GGGATGGGCTTGCC | | V5 tag anti sense |
| Mature peptide and GS linker | CD86 | CD86 MP_F<SEQ ID NO: 19> | TATTTCAATGAGACTGCAGACCT | | NM_175862 (BKU002587) |
| | | CD86 MP_Linker_R <SEQ ID NO: 20> | TCCGCTTCCTCCTCCTCCGCTTCCACCTCC TCCAAAACATGTATCACTTTTGTCGCA | | |
| | CD271 | V5_LNGFR_F <SEQ ID NO: 21> | CCCAACCCCCTGCTGGGCCTGGACAGCAC CAAGGAGGCATGCCCCACAG | | NGFR (Addgene plasmid # 27489) |
| | | LNGFR_Linke r R<SEQ ID NO: 22> | CTCCGCTTCCTCCTCCTCCGCTTCCACCTC CTCCTCGATAAGCTTGGCTGCAGGT | | |
| | CD105 | V5-CD105_F<SE Q ID NO: 23> | CCCAACCCCCTGCTGGGCCTGGACAGCAC CGAAACAGTCCATTGTGACCT | | NM_00111475 3 (BKU008396) |
| | | CD105-GS_R<SEQ ID NO: 24> | CTCCGCTTCCTCCTCCTCCGCTTCCACCTC CTCCTGCCATGCTGCTGGTGGAGC | | |

**[Table 2] Types and sequences of plasmids (Signal peptide; PREY; V5 tag; Transmembrane protein; GS linker and EGFP)**

| Plasmid | Sequence (5'→3') |
|---|---|
| PREY-CD271-EGFP (1701 bp)<SEQ ID NO: 25> | CCATCCTGGTCGAGCTGGACGGCGACGTAAACGGCCACAAGTTCAGCGTGTCCGGC GAGGGCGAGGGCGATGCCACCTACGGCAAGCTGACCCTGAAGTTCATCTGCACCA CCGGCAAGCTGCCCGTGCCCTGGCCCACCCTCGTGACCACCCTGACCTACGGCGTG CAGTGCTTCAGCCGCTACCCCGACCACATGAAGCAGCACGACTTCTTCAAGTCCGC CATGCCCGAAGGCTACGTCCAGGAGCGCACCATCTTCTTCAAGGACGACGGCAACT ACAAGACCCGCGCCGAGGTGAAGTTCGAGGGCGACACCCTGGTGAACCGCATCGA GCTGAAGGGCATCGACTTCAAGGAGGACGGCAACATCCTGGGGCACAAGCTGGAG TACAACTACAACAGCCACAACGTCTATATCATGGCCGACAAGCAGAAGAACGGCA TCAAGGTGAACTTCAAGATCCGCCACAACATCGAGGACGGCAGCGTGCAGCTCGC CGACCACTACCAGCAGAACACCCCCATCGGCGACGGCCCCGTGCTGCTGCCCGACA ACCACTACCTGAGCACCCAGTCCGCCCTGAGCAAAGACCCCAACGAGAAGCGCGA TCACATGGTCCTGCTGGAGTTCGTGACCGCCGCCGGGATCACTCTCGGCATGGACG AGCTGTACAAGTAA |
| PREY -CD86-EGFP (1827 bp)<SEQ ID NO: 26> | AAAGTTCGAAGACATCTTCATGCGACAAAAGTGATACATGTTTTGGAGGAGGTGG AAGCGGAGGAGGAGGAAGCGGAGGAGGAGGTAGCGTGAGCAAGGGCGAGGAGCT GTTCACCGGGGTGGTGCCCATCCTGGTCGAGCTGGACGGCGACGTAAACGGCCAC AAGTTCAGCGTGTCCGGCGAGGGCGAGGGCGATGCCACCTACGGCAAGCTGACCC TGAAGTTCATCTGCACCACCGGCAAGCTGCCCGTGCCCTGGCCCACCCTCGTGACC ACCCTGACCTACGGCGTGCAGTGCTTCAGCCGCTACCCCGACCACATGAAGCAGCA CGACTTCTTCAAGTCCGCCATGCCCGAAGGCTACGTCCAGGAGCGCACCATCTTCT TCAAGGACGACGGCAACTACAAGACCCGCGCCGAGGTGAAGTTCGAGGGCGACAC CCTGGTGAACCGCATCGAGCTGAAGGGCATCGACTTCAAGGAGGACGGCAACATC CTGGGGCACAAGCTGGAGTACAACTACAACAGCCACAACGTCTATATCATGGCCG ACAAGCAGAAGAACGGCATCAAGGTGAACTTCAAGATCCGCCACAACATCGAGGA CGGCAGCGTGCAGCTCGCCGACCACTACCAGCAGAACACCCCCATCGGCGACGGC CCCGTGCTGCTGCCCGACAACCACTACCTGAGCACCCAGTCCGCCCTGAGCAAAGA CCCCAACGAGAAGCGCGATCACATGGTCCTGCTGGAGTTCGTGACCGCCGCCGGG ATCACTCTCGGCATGGACGAGCTGTACAAGTAA |
| PREY-CD 105-EGFP (2814 bp)<SEQ ID NO: 27> | CAGCATGGCAGGAGGAGGTGGAAGCGGAGGAGGAGGAAGCGGAGGAGGAGGTAG CGTGAGCAAGGGCGAGGAGCTGTTCACCGGGGTGGTGCCCATCCTGGTCGAGCTG GACGGCGACGTAAACGGCCACAAGTTCAGCGTGTCCGGCGAGGGCGAGGGCGATG CCACCTACGGCAAGCTGACCCTGAAGTTCATCTGCACCACCGGCAAGCTGCCCGTG CCCTGGCCCACCCTCGTGACCACCCTGACCTACGGCGTGCAGTGCTTCAGCCGCTA CCCCGACCACATGAAGCAGCACGACTTCTTCAAGTCCGCCATGCCCGAAGGCTACG TCCAGGAGCGCACCATCTTCTTCAAGGACGACGGCAACTACAAGACCCGCGCCGA GGTGAAGTTCGAGGGCGACACCCTGGTGAACCGCATCGAGCTGAAGGGCATCGAC TTCAAGGAGGACGGCAACATCCTGGGGCACAAGCTGGAGTACAACTACAACAGCC ACAACGTCTATATCATGGCCGACAAGCAGAAGAACGGCATCAAGGTGAACTTCAA GATCCGCCACAACATCGAGGACGGCAGCGTGCAGCTCGCCGACCACTACCAGCAG AACACCCCCATCGGCGACGGCCCCGTGCTGCTGCCCGACAACCACTACCTGAGCAC CCAGTCCGCCCTGAGCAAAGACCCCAACGAGAAGCGCGATCACATGGTCCTGCTG GAGTTCGTGACCGCCGCCGGGATCACTCTCGGCATGGACGAGCTGTACAAGTAA |

### (Measurement of transfection efficiency)

PREY transfection efficiency was compared among the test candidates (CD86, CD105, and CD271) of transmembrane proteins with two types of MSCs (ASC and BMSC) one day after transfection by flow cytometry using FACSCanto (BD Biosciences, CA) with quantitative analysis. Therefore, cells were immunostained with an anti-v5 tag primary antibody (ab27671, Abcam, MA) and an Alexa Fluor 647-conjugated secondary antibody (Jackson Immuno Research, PA). To evaluate plasmid dose-dependent apoptosis, transfected ASCs and BMSCs were harvested 30 minutes after transfection, immunostained with Alexa Fluor 488-conjugated annexin V (Thermo Fisher Scientific, CA), and they were subjected to flow cytometry. The number of viable cells was also counted by trypan blue staining one day after transfection.

### (NV extrusion)

Three days after transfection, human ASCs (Promocell, Germany), BMSCs (Lonza, Switzerland), and PMSCs were washed twice with PBS, and detached with 0.25% trypsin/EDTA. Subsequently, to produce nanovesicles, 1×10⁶ cells/mL of the cell suspension in PBS was extruded 6 times sequentially with polycarbonate membrane filters with pore sizes of 10 µm, 5 µm, and 400 nm (Whatman, UK) using an extrusion kit (Avanti Polar Lipids, AL). The NVs were collected by centrifugation at 15,000 g for 30 minutes. Subsequently, the pellet was re-suspended in PBS, filtered using a 0.20-µm syringe filter (Avantec, Japan), and the resulting product was stored at -70 °C until use. The size and morphology of PMSC-NVs were determined by transmission electron microscope (TEM; JEM-F200, JEOL, Japan) and dynamic light scattering (DLS; ELS-1000ZS, Otsuka Electronics, Japan).

### (Measurement of in vitro anti-inflammatory effects)

RAW264.7 cells were seeded on a 24-well plate (5×10⁵ cells/well). The pro-inflammatory activation of the RAW264.7 cells was induced in ASC-NV or BMSC-NV (10 µg/mL) after 24-hour LPS (Sigma-Aldrich; 100 ng/mL) treatments. To visualize cellular uptake, the RAW264.7 cells and NVs were labeled with DiO and DiI (Invitrogen, CA), respectively, and imaged using a confocal microscope (LSM780; Zeiss, Germany). For qRT-PCR analysis, the cells were collected 24 hours after NV treatments. Primer sequences of IL-10, IL-1β, IL-6, and TNF-α are listed in Table 3. For cytokine analysis using a mouse inflammation antibody array (ab133999, Abcam), a cell supernatant was collected according to the manufacturer's instructions. The anti-phagocytic effects of MSC-NVs were measured according to the manufacturer's instructions using a Vybrant^{™} Phagocytosis Assay Kit (V6694, Molecular Probes, OR). Images were obtained using a confocal microscope, and a fluorescent intensity was measured using a Varioskan^{™} LUX multimode microplate reader (Thermo Fisher Scientific, MA).

**[Table 3]**

| **Target gene** | **Primer sequence (5'→3')** | | **SEQ ID NO.** |
|---|---|---|---|
| GAPDH | Forward | ATG TGT CCG TCG TGG ATC TGA | 28 |
| | Reverse | TGC CTG CTT CAC CAC CTT CT | 29 |
| IL-10 | Forward | ACT GGC ATG AGG ATC AGC AG | 30 |
| | Reverse | CTC CTT GAT TTC TGG GCC AT | 31 |
| IL-1β | Forward | GCC ACC TTT TGA CAG TGA TGA G | 32 |
| | Reverse | ATC AGG ACA GCC CAG GTC AA | 33 |
| IL-13 | Forward | ATG GCC TCT GTA ACC GCA AG | 34 |
| | Reverse | TCC TCA TTA GAA GGG GCC GT | 35 |
| TNF-α | Forward | CCT GTA GCC CAC GTC GTA GC | 36 |
| | Reverse | AGC AAT GAC TCC AAA GTA GAC C | 37 |
| VCAM1 | Forward | AGT TGG GGA TTC GGT TGT TC | 38 |
| | Reverse | CAT TCC TTA CCA CCC CAT TG | 39 |
| E-selectin | Forward | CCA GAA TGG CGT CAT GGA | 40 |
| | Reverse | TAA AGC CCT CAT TGC ATT GA | 41 |
| ICAM1 | Forward | CAA TTT CTC ATG CCG CAC AG | 42 |
| | Reverse | AGC TGG AAG ATC GAA AGT CCG | 43 |

### (Evaluation of in vitro pro-EC recovery effects)

iMAECs (ATCC, VA) were seeded on a 24-well plate (1×10⁵ cells/well) and then treated with LPS (100 ng/mL) for 24 hours. Subsequently, the cells were additionally treated with ASC-NVs, BMSC-NVs or PMSC-NVs (10 µg/mL) for 24 hours. For visualization of cell uptake, the iMAECs and NVs were labeled with DiO and DiI, respectively, and visualized using a confocal microscope. For qRT-PCR analysis, iMAECs were collected 24 hours after NV treatments. Primer sequences of E-selectin, ICAM-1, and VCAM-1 are listed in Table 3. The iMAECs were immunostained with a VCAM-1 antibody (ab134047, Abcam) and imaged using a confocal microscope with quantitative analysis by ImageJ. HUVECs (Lonza) were treated with NVs (10 µg/mL) and CyA (25 µg/mL; Santa Cruz Biotechnology, CA), and they were cultured on Matrigel (BD Biosciences, MA) for 2 hours or 24 hours, followed by measuring the pro-EC recovery effects caused by anti-angiogenesis and angiorrhexis. Images were obtained using a confocal microscope and quantified using ImageJ.

### (Murine PCL model)

All animal studies were carried out by procedures (2018-0044) approved by the Institutional Animal Care and Use Committee (IACUC) of the College of Medicine of Yonsei University, Republic of Korea. The surgical procedure was performed with 6-week-old male Balb/c (Orient Bio Inc, Korea) or KOR-ApoE (shl)(SLC, Japan) mice. For PCL surgery, the animals were anesthetized by intraperitoneal injection of a mixture of xylazine (10 mg/kg) and zoletil (50 mg/kg). The neck of each animal was shaved and disinfected with betadine. Subsequently, a midline incision was performed (5 mm). After LCA exposure, three (ECA, ICA and OA) of the four branches of the LCA were ligated with 10-0 polyamide suture, and the STA was left unligated. Subsequently, the incision was closed with 6-0 silk suture. In addition, the mice were monitored and fed an atherosclerotic diet (Research Diets, NJ), followed by intravenously administering MSC-NVs, PMSCs, or PMSC-NVs three days after ligation.

### (Measurement of targeting efficiency and anti-atherosclerotic effects in murine PCL model)

The *in vivo* PMSC-NV targeting efficiency of disturbed blood flow sites was measured in murine PCL models through IVIS imaging (PerkinElmer, WA) and histological analysis 24 hours after injection of the test groups. The MSC and NV groups were labeled with VivoTrack 680 (PerkinElmer) for 30 minutes and injected into the PCL models. Subsequently, IVIS imaging was performed under inhalational anesthetization with isoflurane. Afterwards, the mice were sacrificed, and their LCAs, RCAs and major organs were harvested for ex-vivo IVIS imaging and histological analysis. Tissue sections of the LCAs and RCAs were immunostained with an anti-filamin A antibody (ab51217, Abcam), and the corresponding fluorescence intensity was quantified using ImageJ software. Tissue sections were also stained with H&E, or immunostained with an anti-CD68 antibody (ab125212, Abcam) and an anti-VCAM-1 antibody (ab134047, Abcam). Neointima structure factors (area ratio of neointima to neointima+ lumen, area ratio of neointima to media, and neointima area) or the corresponding fluorescence intensities were quantitatively analyzed using ImageJ.

### (Measurement of targeting efficiency in porcine PCL model)

PCL surgery was performed on female Yorkshire pigs with a weight of 25 to 30 kg (XP bio, Republic of Korea) according to a previous study. The pigs were subjected to intramuscular injection with atropine (0.04 mg/kg), xylazine (2 mg/kg), and azaperone (2 mg/kg) as premedication. The pigs were anesthetized with alfaxan (1 mg/kg) and maintained in this state by endotracheal intubation of 2% isoflurane during surgery. The neck was disinfected using betadine and then a midline skin incision was performed. A sterilized stainless-steel rod (outer diameter=0.9 mm) was placed as a spacer on the LCA and ligated together with 5-0 silk suture. The rod was sequentially removed, and the incision was closed by suturing, resulting in 80% occlusion of a carotid artery. The RCA was left unligated as a normal group. A blood flow pattern was observed using ultrasound (S22V; SonoScape Medical Corp., China). At three days after ligations, Vivotrack680-labeled MSC-NVs or PMSC-NVs were intravenously injected into an ear vein (1 mg/pig), and the pigs were sacrificed on day 1 or 21 after injection. For ex-vivo IVIS and histological analysis, RCAs, LCAs, and aortic arches were collected. These tissue sections were immunostained with an anti-filamin A antibody and an anti-CD31 antibody (sc-1506, Santa Cruz Biotechnology, CA), followed by fluorescence imaging with ImageJ analysis.

### (Measurement of human EC targeting efficiency in in vitro blood flow model)

As previously described (Sei, Y. J. et al. Sci Rep 7, 10019, 2017), a microfluidic device was produced with polydimethylsiloxane (PDMS, Dow Corning, MI) by soft lithography, bonded with glass coverslips (VWR, PA), and placed in a polystyrene box (Ted Pella Inc., CA). The devices were sterilized with 70% ethanol and washed with PBS. Channels were then coated with 50 µg/mL of collagen I (Corning, MA) at 37 °C for 1 hour. Human coronary artery endothelial cells (hCAECs; Lonza) or human aortic endothelial cells (hAECs; Lonza) were seeded into the channel for 12 hours (2×10⁷ cells/mL). The outlet of each device was connected to a PhD Ultra syringe pump (Harvard Apparatus, MA) to create a normal medium flow or a disturbed blood flow. To create the normal laminal flow, the medium was perfused with a shear stress of 10 dyne/cm² at 22.5 µL/min. The disturbed blood flow was generated by repeated cycles of injecting and removing the medium at 22.5 µL/min (10 dyne/cm²) and 20 µL/min (9 dyne/cm²), respectively. After the human ECs were exposed to one of the flow types, NVs (10 µg/mL) were perfused into the channels at 37 °C for 1 hour. NV uptake into human ECs in each test group was quantitatively analyzed using ImageJ.

### (qRT-PCR and miRNA array)

Total RNA was extracted from each sample using a 1 mL TRIzol reagent (Invitrogen) according to the manufacturer's instructions. The RNA was dissolved in diethyl pyrocarbonate (DEPC) water, and cDNA was synthesized using AccuPower CycleScript RT Premix (Bioneer, Republic of Korea). Subsequently, PCR was performed with SYBR Green PCR mix (Thermo Fisher Scientific) in a StepOnePlus real-time PCR system (Applied Biosystems, CA). Glyceraldehyde 3-phosphate dehydrogenase (GAPDH) was provided as a housekeeping gene, and the gene expression of each marker was measured using the relative quantification method (2^{-ΔΔCt}). Primer sequences are listed in Table 3. The dissolved RNA was profiled using a miRNA array (GeneChip 4.0 microRNA Microarray; Affimetrix, Japan) according to the manufacturer's instructions.

### (Immunofluorescence staining of cells and tissue)

Cell samples were fixed with 4% paraformaldehyde (Sigma-Aldrich) for 10 minutes, tissue samples were fixed with 10% paraformaldehyde for 3 days, and both were performed at room temperature. The fixed samples were washed with PBS, and embedded in paraffin to make tissue sections. Subsequently, the sections were hydrated using a series of xylene and ethanol solutions (100%, 95%, 80% and 70% v/v in distilled water) and treated with a pepsin reagent (Sigma-Aldrich) for 30 minutes at 37 °C for antigen retrieval. The tissue sections were then treated with a blocking solution (5% bovine serum albumin (Millipore, MD) + 0.3% Triton X-100 (Sigma-Aldrich)) at room temperature for 1 hour. The primary antibodies are an anti-v5 tag antibody (ab27671, Abcam), anti-VCAM-1 (ab134047, Abcam), anti-CD68 (ab125212, Abcam), anti-Filamin A (ab51217, Abcam), and anti-CD31 (sc-1506, Santa Cruz Biotechnology, CA, USA). These antibodies were treated in PBS with 1: 100 dilutions, and subsequent secondary antibodies were treated with PBS in 1:200 dilutions. The secondary antibodies are an anti-mouse antibody conjugated to Alexa Fluor 594, an anti-rabbit antibody conjugated to Alexa Fluor 594, an anti-rabbit antibody conjugated to Alexa Fluor 488 and an anti-goat antibody conjugated to Alexa Fluor 488 (all from Jackson Laboratories). Subsequently, the samples were mounted and counterstained with a mounting solution containing 4',6-diamidino-2-phenylindole (DAPI, Vector Laboratories, CA) to visualize the cell nuclei.

### (Western blotting)

The samples were lysed with RIPA buffer (Sigma-Aldrich) to obtain total proteins, and the concentrations of the proteins were measured using a Bradford assay (Sigma-Aldrich). Protein extracts were run on a 10%(w/v) SDS-PAGE gel by electrophoresis and then transferred to a nitrocellulose membrane. The membrane was blocked in TBST (20 mM Tris, 0.9% NaCl, 0.1% Tween 20, pH 7.4) with 5%(w/v) skim milk and then incubated with primary antibodies such as anti-v5 tag (ab27671, Abcam), anti-CD271 (345102, Biolegend, CA), anti-GFP (ab32146, Abcam), anti-CD9(ab92726, Abcam), and anti-β-actin (ab8227, Abcam). Subsequently, secondary antibodies such as goat anti-mouse IgG (H+L)-HRP conjugated and goat anti-rabbit IgG (H+L)-HRP conjugated antibodies (all from Vector Laboratories) were applied according to the manufacturer's instructions. The signals were visualized using a CL Plus Western Blotting Detection Kit (Amersham Biosciences, UK) according to the manufacturer's instructions and analyzed using a LAS-3000 image reader (Fujifilm, Japan).

### (Statistical analysis)

Quantitative data is expressed as mean ± standard deviation (stdev). The results are statistically analyzed through by one-way ANOVA by a Tukey's significant difference post hoc test using SigmaPlot 12.0 (Systat Software, Inc., California, USA).

### <Experimental Example 1> RPEY display at outside of membrane

To enable NVs to target disturbed blood flow sites, a targeting peptide PREY was externally displayed by expressing a specifically-designed plasmid across the MSC membrane (PMSCs: PREY-expressing human MSCs). The plasmid was designed to express a PREY extracellular domain with a signal peptide at the N-terminus so that the signal peptide can induce the outward localization of the PREY-extracellular domain from the MSC membrane and deactivate the induction signal by cleavage.

In addition, the ability of three transmembrane proteins (TMPs), that is, the exosome marker (CD86) and MSC markers (CD105 and CD271), for improving PREY expression was tested. In this way, the ability of PREY to search for and target disturbed blood flow sites was maximized. PREY was linked with V5 tag and then with GFP (external N-terminus-promoter-signal peptide-PREY-V5 tag-TMP-GFP-internal C-terminus) to confirm the localization and the expression levels of PREY outside and inside the cell membrane (FIGS. 2A and 3).

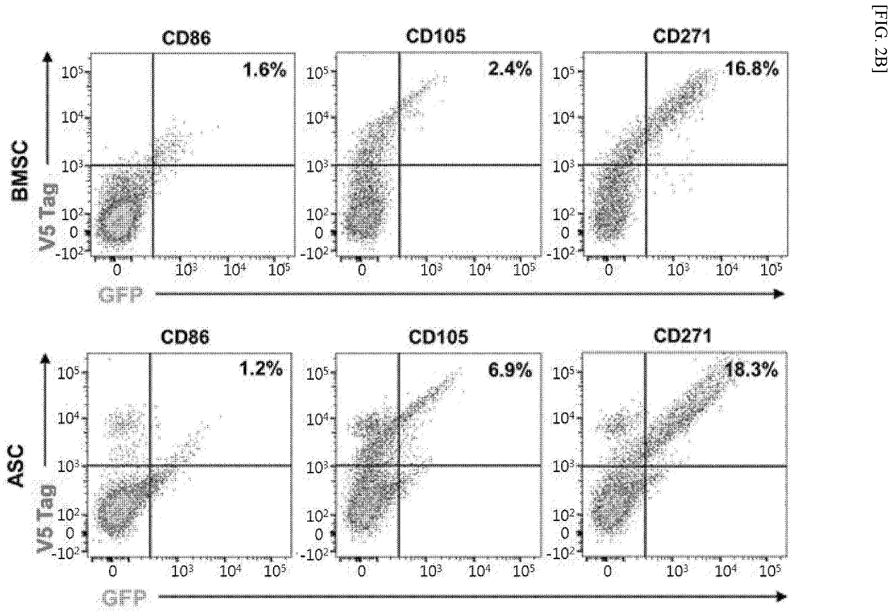

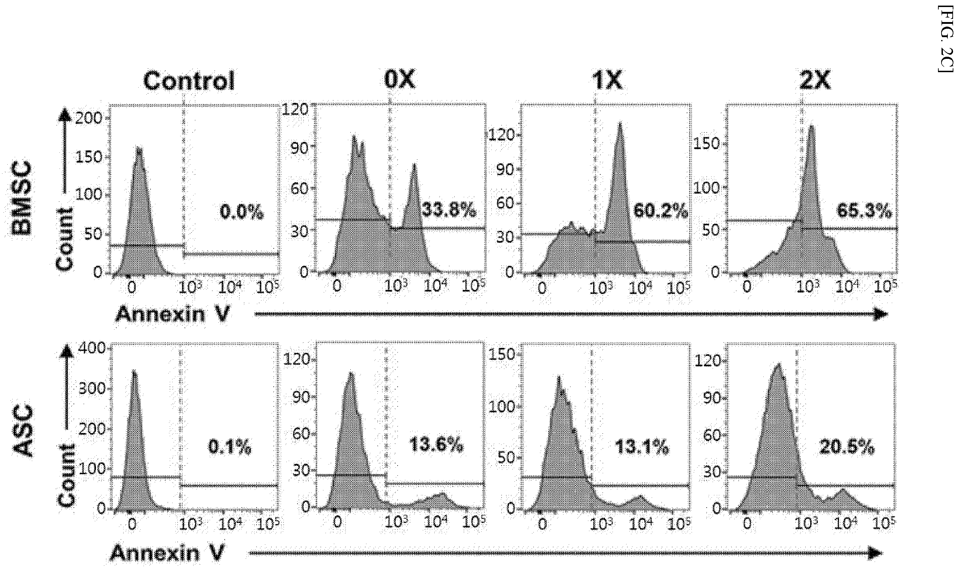

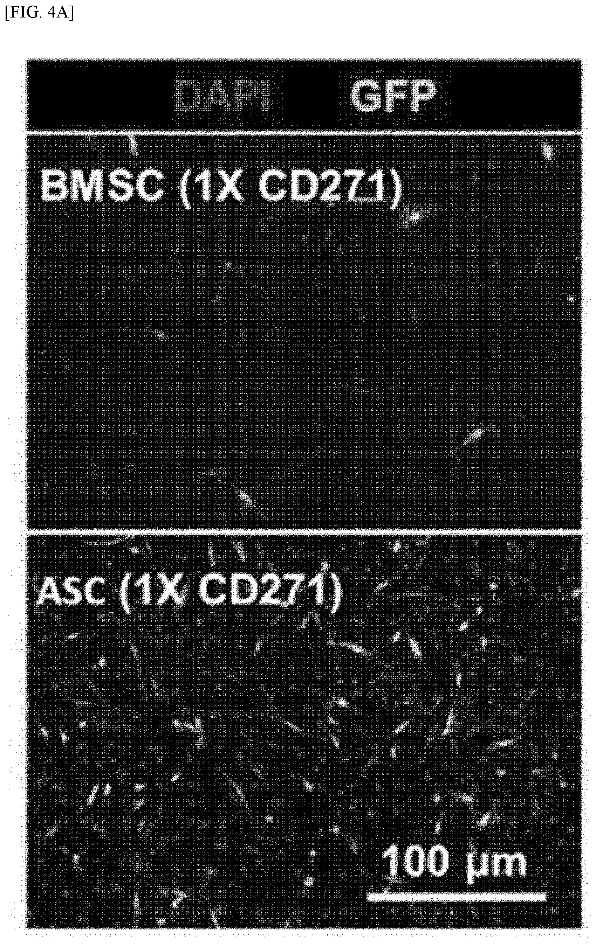

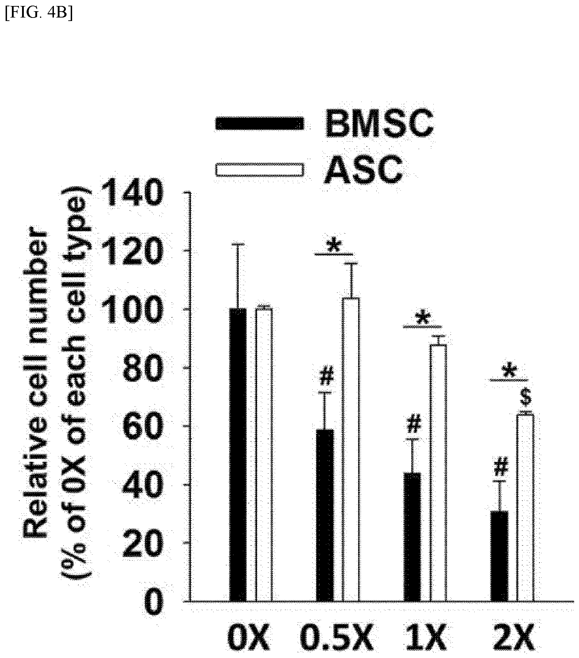

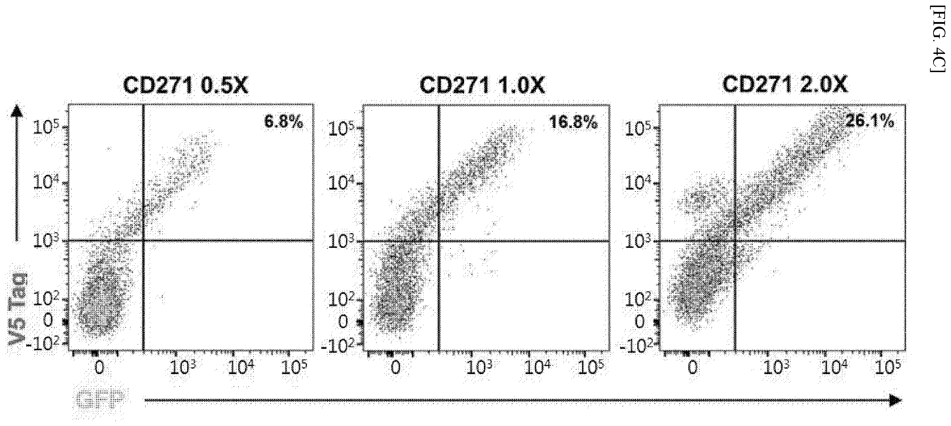

Among the test groups having the three transmembrane proteins, the expression level of PREY-CD271 (nerve growth factor receptor, NGFR) was higher than those of PREY-CD86 and PREY-CD105 in both adipose-derived stem cells (ASCs) and bone marrow-derived stem cells (BMSCs), showing excellent transfection efficiency (FIGS. 2B, and 4A to 4C). When the dose of the PREY-CD271 plasmid was increased, the expression level increased in a dose-dependent manner, whereas cell viability was dramatically reduced after 2X-dose transfection (FIGS. 2C, and 4A to 4C). Accordingly, in consideration of the balance with cell viability, the plasmid dose was determined to be 1 × (1 µg PREY-plasmid per 1×10⁶ cells). Comparing the number of dead cells using Annexin V+, there were more viable ASCs than BMSCs after transfection, indicating ASCs are a more suitable source for PREY transfection (FIGS. 2C, and 4A to 4C).

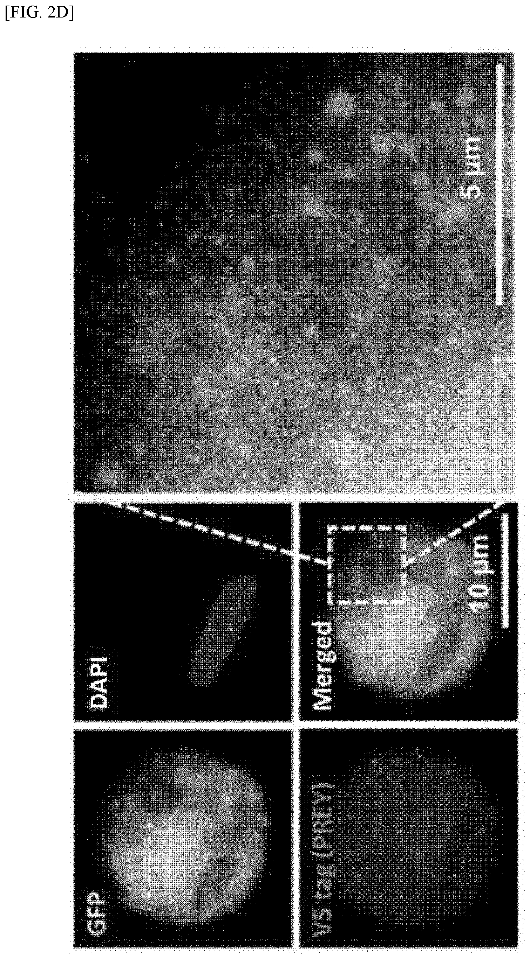

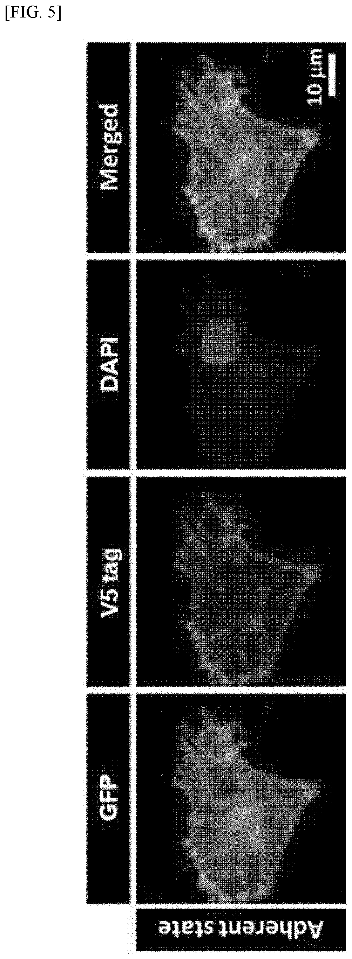

To verify the external display of PREY on the MSC membrane, the V5 tag was immunostained when placed next to the PREY in the sequence (external N-terminus-promoter-signal peptide-PREY-V5-TMP-GFP-internal C-terminus). Since neither of the two types worked to clearly obtain both information types, adhered (FIG. 5) and suspended (FIGS. 2D, and FIGS. 1A to 1C) forms of PMSCs were used to determine the expression level and location of PREY.

In the adhered form, PREY was highly expressed along the cell membrane as shown by the v5 tag and GFP signal, indicating successful transfection. When suspended forms were formed by inserting cells into a gelatin hydrogel, the confocal images showed the appearance of the v5 tag (red) and GFP (green) signals outside and inside the boundary of the MSC membrane, respectively, confirming the external PREY display on the cell membrane.

### <Experimental Example 2> Extrusion and characterization of nanovesicles

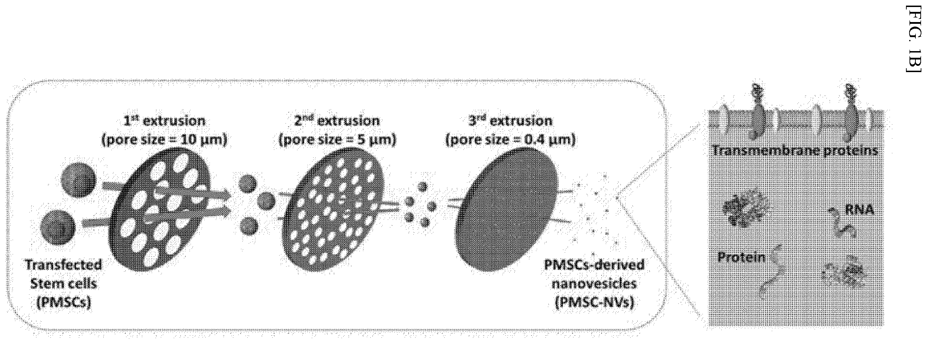

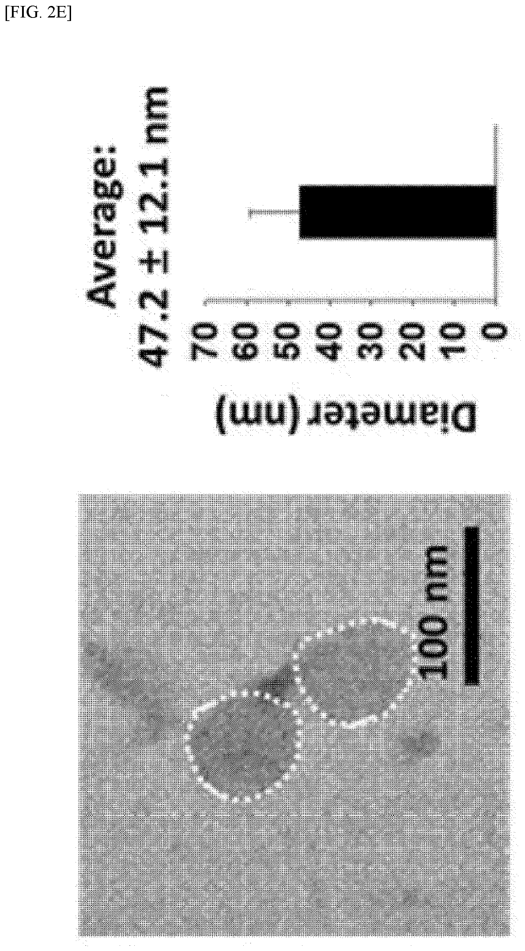

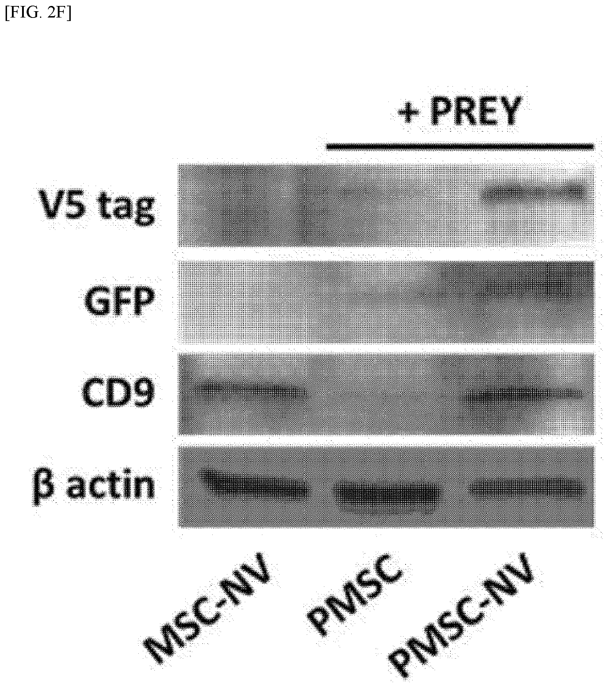

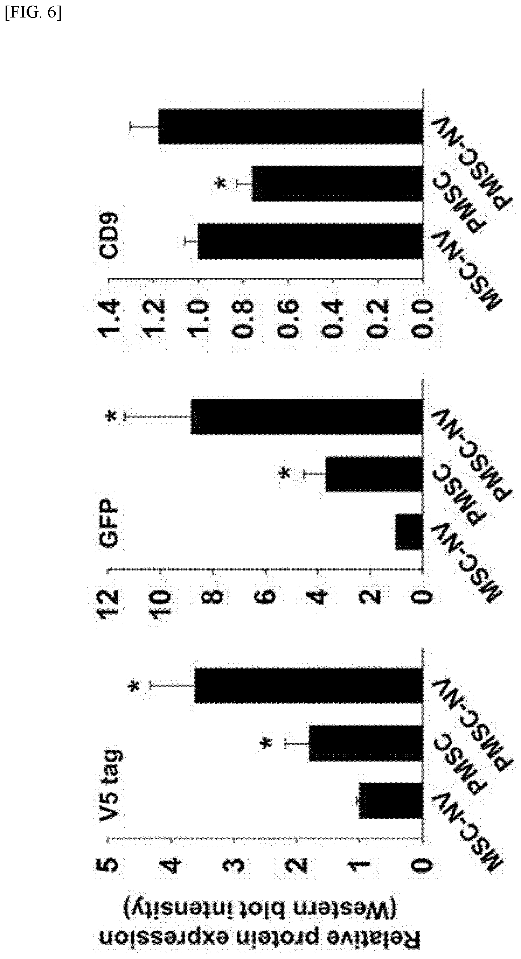

To produce PMSC-NVs, PMSCs were sequentially extruded through a series of polycarbonate micropore membranes while gradually decreasing the pore diameters from 10 to 5 µm and finally to 0.4 µm (FIG. 1B). A TEM result (FIG. 2E) shows a uniform distribution of PMSC-NV diameters with an average of 47.2±12.1 nm. In addition, the intact preservation of the PREY-peptide (GFP and v5 tag) and exosome characteristics (CD9) during transfection and NV extrusion were assessed by western blotting (FIGS. 2F and 6).

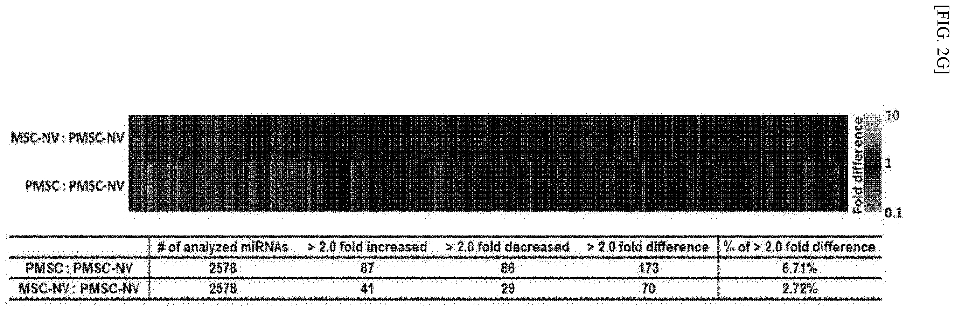

Next, comparing the intracellular components in MSC-NVs, PMSCs and PMSC-NVs, the preservation of intracellular components was confirmed after PREY transfection and NV extrusion. miRNAs are provided as important therapeutic agents, and most of them are delivered by exosomes. Since NVs are inherently similar to exosomes, the miRNA contents of PMSC-NVs, MSC-NVs, and PMSCs were profiled by miRNA array (FIG. 2G and Tables 4 and 5, and FIG. 6). The transfection (MSC-NV vs, PMSC-NV) and extrusion (PMSC vs. PMSC-NV) processes change the expression levels at least two-fold with respect to 2.72 and 6.71% of the total miRNAs, proving the preservation of intracellular content during their production. Among anti-atherosclerotic miRNAs, miR-21, miR-132, miR-10, miR-146, miR-143, and let 7 levels increased during extrusion, but the expression levels of other miRNAs were maintained during NV production from MSCs.

**[Table 4]**

| Name of miRNA | Change rate | Regulation pattern | Name of miRNA | Change rate | Regulation pattern |
|---|---|---|---|---|---|
| hsa-miR-21-5p | 17.321 | up-regulation | hsa-miR-3195 | 5.080 | down-regulation |
| hsa-miR-140-5p | 10.011 | up-regulation | hsa-miR-106b-3p | 4.296 | down-regulation |
| hsa-miR-7641 | 9.352 | up-regulation | hsa-miR-3663-3p | 3.766 | down-regulation |
| hsa-miR-337-5p | 8.283 | up-regulation | hsa-miR-345-5p | 3.375 | down-regulation |
| hsa-miR-4443 | 8.228 | up-regulation | hsa-miR-3178 | 3.057 | down-regulation |
| hsa-miR-4726-5p | 7.288 | up-regulation | hsa-miR-3687 | 3.035 | down-regulation |
| hsa-miR-132-3p | 4.383 | up-regulation | hsa-miR-8063 | 2.958 | down-regulation |
| hsa-miR-376c-3p | 4.236 | up-regulation | hsa-miR-4634 | 2.843 | down-regulation |
| hsa-miR-19b-3p | 3.558 | up-regulation | hsa-miR-7112-5p | 2.840 | down-regulation |
| hsa-miR-503-5p | 3.481 | up-regulation | hsa-miR-663b | 2.661 | down-regulation |
| hsa-miR-196a-5p | 3.366 | up-regulation | hsa-miR-3124-5p | 2.549 | down-regulation |
| hsa-miR-15a-5p | 3.178 | up-regulation | hsa-miR-4497 | 2.523 | down-regulation |
| hsa-miR-143-3p | 3.091 | up-regulation | hsa-miR-7975 | 2.505 | down-regulation |
| hsa-miR-143-5p | 2.750 | up-regulation | hsa-miR-3679-5p | 2.490 | down-regulation |
| hsa-miR-629-5p | 2.689 | up-regulation | hsa-miR-6127 | 2.450 | down-regulation |
| hsa-miR-194-5p | 2.549 | up-regulation | hsa-miR-6871-5p | 2.442 | down-regulation |
| hsa-miR-30b-5p | 2.521 | up-regulation | hsa-miR-4486 | 2.405 | down-regulation |
| hsa-miR-27a-3p | 2.467 | up-regulation | hsa-miR-4750-5p | 2.278 | down-regulation |
| hsa-miR-3131 | 2.466 | up-regulation | hsa-miR-6509-5p | 2.221 | down-regulation |
| hsa-miR-7109-5p | 2.442 | up-regulation | hsa-miR-4649-5p | 2.158 | down-regulation |
| hsa-miR-30a-5p | 2.424 | up-regulation | hsa-miR-6820-5p | 2.156 | down-regulation |
| hsa-miR-152-3p | 2.407 | up-regulation | hsa-miR-15b-5p | 2.149 | down-regulation |
| hsa-miR-6837-5p | 2.379 | up-regulation | hsa-miR-505-5p | 2.145 | down-regulation |
| hsa-miR-1247-3p | 2.370 | up-regulation | hsa-miR-378a-3p | 2.136 | down-regulation |
| hsa-miR-379-5p | 2.254 | up-regulation | hsa-miR-1343-5p | 2.126 | down-regulation |
| hsa-miR-199a-5p | 2.248 | up-regulation | hsa-miR-30d-5p | 2.097 | down-regulation |
| hsa-let-7f- 5p | 2.246 | up-regulation | hsa-miR-486-5p | 2.089 | down-regulation |
| hsa-miR-6876-5p | 2.239 | up-regulation | hsa-miR-1469 | 2.082 | down-regulation |
| hsa-miR-642a-3p | 2.221 | up-regulation | hsa-miR-6831-5p | 2.025 | down-regulation |
| hsa-miR-1260b | 2.195 | up-regulation | | | |
| hsa-miR-210-3p | 2.184 | up-regulation | | | |
| hsa-let-7i-5p | 2.181 | up-regulation | | | |
| hsa-miR-452-5p | 2.157 | up-regulation | | | |
| hsa-miR-27b-3p | 2.131 | up-regulation | | | |
| hsa-miR-4521 | 2.128 | up-regulation | | | |
| hsa-miR-3201 | 2.119 | up-regulation | | | |
| hsa-miR-371b-5p | 2.084 | up-regulation | | | |
| hsa-miR-654-3p | 2.081 | up-regulation | | | |
| hsa-miR-4487 | 2.049 | up-regulation | | | |
| hsa-miR-22-3p | 2.039 | up-regulation | | | |
| hsa-let-7c-5p | 2.022 | up-regulation | | | |

**[Table 5] At least 2-fold miRNA change by extrusion process (PMSC vs. PMSC-NV)**

| Name of miRNA | Change rate | Regulation pattern | Name of miRNA | Change rate | Regulation pattern |
|---|---|---|---|---|---|
| hsa-miR-21-5p | 11.055 | up-regulation | hsa-miR-3162-5p | 8.520 | down-regulation |
| hsa-miR-132-3p | 10.252 | up-regulation | hsa-miR-4298 | 8.288 | down-regulation |
| hsa-miR-4521 | 9.785 | up-regulation | hsa-miR-6778-5p | 6.988 | down-regulation |
| hsa-miR-337-5p | 7.805 | up-regulation | hsa-miR-1973 | 5.582 | down-regulation |
| hsa-miR-1260b | 7.468 | up-regulation | hsa-miR-3911 | 5.221 | down-regulation |
| hsa-miR-27b-5p | 7.120 | up-regulation | hsa-miR-7107-5p | 5.104 | down-regulation |
| hsa-miR-1260a | 7.045 | up-regulation | hsa-miR-6782-5p | 4.878 | down-regulation |
| hsa-miR-99b-3p | 6.854 | up-regulation | hsa-miR-7847-3p | 4.844 | down-regulation |
| hsa-miR-425-3p | 5.547 | up-regulation | hsa-miR-6831-5p | 4.763 | down-regulation |
| hsa-miR-10a-5p | 5.071 | up-regulation | hsa-miR-6726-5p | 4.708 | down-regulation |
| hsa-miR-214-5p | 4.763 | up-regulation | hsa-miR-6068 | 4.695 | down-regulation |
| hsa-miR-7975 | 4.746 | up-regulation | hsa-miR-4656 | 4.567 | down-regulation |
| hsa-miR-15a-5p | 4.745 | up-regulation | hsa-miR-6812-5p | 4.230 | down-regulation |
| hsa-miR-378a-3p | 4.567 | up-regulation | hsa-miR-485-3p | 4.208 | down-regulation |
| hsa-miR-487a-5p | 4.512 | up-regulation | hsa-miR-8089 | 4.136 | down-regulation |
| hsa-miR-27a-5p | 4.421 | up-regulation | hsa-miR-4534 | 4.126 | down-regulation |
| hsa-miR-140-5p | 4.360 | up-regulation | hsa-miR-3135b | 4.065 | down-regulation |
| hsa-miR-500a-5p | 4.265 | up-regulation | hsa-miR-6813-5p | 4.014 | down-regulation |
| hsa-miR-7641 | 3.989 | up-regulation | hsa-miR-6808-5p | 3.966 | down-regulation |
| hsa-miR-381-3p | 3.901 | up-regulation | hsa-miR-4732-5p | 3.920 | down-regulation |
| hsa-miR-378h | 3.799 | up-regulation | hsa-miR-7111-5p | 3.902 | down-regulation |
| hsa-miR-30a-5p | 3.795 | up-regulation | hsa-miR-6848-5p | 3.889 | down-regulation |
| hsa-miR-629-5p | 3.739 | up-regulation | hsa-miR-4433-3p | 3.768 | down-regulation |
| hsa-miR-452-5p | 3.706 | up-regulation | hsa-miR-4433b-3p | 3.750 | down-regulation |
| hsa-miR-196a-5p | 3.677 | up-regulation | hsa-miR-6086 | 3.710 | down-regulation |
| hsa-miR-4487 | 3.663 | up-regulation | hsa-miR-6165 | 3.646 | down-regulation |
| hsa-miR-29b-1-5p | 3.659 | up-regulation | hsa-miR-5189-3p | 3.630 | down-regulation |
| hsa-miR-4454 | 3.457 | up-regulation | hsa-miR-1229-5p | 3.532 | down-regulation |
| hsa-miR-93-3p | 3.367 | up-regulation | hsa-miR-6763-5p | 3.513 | down-regulation |
| hsa-miR-154-5p | 3.333 | up-regulation | hsa-miR-619-5p | 3.467 | down-regulation |
| hsa-miR-146a-5p | 3.332 | up-regulation | hsa-miR-6861-5p | 3.457 | down-regulation |
| hsa-miR-501-5p | 3.243 | up-regulation | hsa-miR-150-3p | 3.302 | down-regulation |
| hsa-miR-654-3p | 3.232 | up-regulation | hsa-miR-3687 | 3.261 | down-regulation |
| hsa-miR-193b-5p | 3.220 | up-regulation | hsa-miR-5739 | 3.122 | down-regulation |
| hsa-miR-18a-5p | 3.208 | up-regulation | hsa-miR-1247-3p | 3.119 | down-regulation |
| hsa-miR-339-3p | 3.043 | up-regulation | hsa-miR-6820-5p | 3.041 | down-regulation |
| hsa-miR-542-5p | 3.014 | up-regulation | hsa-miR-6716-5p | 3.038 | down-regulation |
| hsa-miR-299-3p | 3.005 | up-regulation | hsa-miR-6775-5p | 2.971 | down-regulation |
| hsa-miR-151a-3p | 2.986 | up-regulation | hsa-miR-4749-5p | 2.968 | down-regulation |
| hsa-miR-199a-5p | 2.942 | up-regulation | hsa-miR-3679-5p | 2.960 | down-regulation |
| hsa-miR-30b-5p | 2.891 | up-regulation | hsa-miR-4728-5p | 2.921 | down-regulation |
| hsa-miR-382-5p | 2.879 | up-regulation | hsa-miR-6769b-5p | 2.822 | down-regulation |
| hsa-miR-379-5p | 2.842 | up-regulation | hsa-miR-7106-5p | 2.805 | down-regulation |
| hsa-miR-187-5p | 2.797 | up-regulation | hsa-miR-7845-5p | 2.794 | down-regulation |
| hsa-miR-376c-3p | 2.795 | up-regulation | hsa-miR-4669 | 2.783 | down-regulation |
| hsa-miR-4443 | 2.701 | up-regulation | hsa-miR-4459 | 2.761 | down-regulation |
| hsa-let-7i-5p | 2.680 | up-regulation | hsa-miR-937-5p | 2.730 | down-regulation |
| hsa-miR-31-3p | 2.618 | up-regulation | hsa-miR-6776-5p | 2.730 | down-regulation |
| hsa-miR-378c | 2.604 | up-regulation | hsa-miR-6819-5p | 2.668 | down-regulation |
| hsa-miR-143-3p | 2.576 | up-regulation | hsa-miR-8063 | 2.590 | down-regulation |
| hsa-miR-99a-5p | 2.574 | up-regulation | hsa-miR-1202 | 2.585 | down-regulation |
| hsa-miR-34a-5p | 2.543 | up-regulation | hsa-miR-6771-5p | 2.573 | down-regulation |
| hsa-miR-210-3p | 2.533 | up-regulation | hsa-miR-7114-5p | 2.557 | down-regulation |
| hsa-miR-93-5p | 2.523 | up-regulation | hsa-miR-6127 | 2.557 | down-regulation |
| hsa-miR-22-3p | 2.523 | up-regulation | hsa-miR-6871-5p | 2.440 | down-regulation |
| hsa-miR-320e | 2.515 | up-regulation | hsa-miR-6743-5p | 2.434 | down-regulation |
| hsa-miR-345-5p | 2.510 | up-regulation | hsa-miR-5006-5p | 2.415 | down-regulation |
| hsa-miR-493-3p | 2.508 | up-regulation | hsa-miR-663b | 2.410 | down-regulation |
| hsa-miR-411-5p | 2.503 | up-regulation | hsa-miR-4687-3p | 2.409 | down-regulation |
| hsa-miR-28-3p | 2.489 | up-regulation | hsa-miR-197-3p | 2.387 | down-regulation |
| hsa-miR-130b-3p | 2.487 | up-regulation | hsa-miR-149-3p | 2.370 | down-regulation |
| hsa-miR-362-5p | 2.464 | up-regulation | hsa-miR-7162-3p | 2.359 | down-regulation |
| hsa-miR-130a-3p | 2.388 | up-regulation | hsa-miR-6799-5p | 2.342 | down-regulation |
| hsa-miR-320d | 2.364 | up-regulation | hsa-miR-6779-5p | 2.340 | down-regulation |
| hsa-miR-19b-3p | 2.336 | up-regulation | hsa-miR-6794-5p | 2.340 | down-regulation |
| hsa-miR-3651 | 2.333 | up-regulation | hsa-miR-4507 | 2.324 | down-regulation |
| hsa-miR-140-3p | 2.320 | up-regulation | hsa-miR-6132 | 2.316 | down-regulation |
| hsa-miR-27a-3p | 2.299 | up-regulation | hsa-miR-6785-5p | 2.291 | down-regulation |
| hsa-miR-425-5p | 2.269 | up-regulation | hsa-miR-7112-5p | 2.279 | down-regulation |
| hsa-miR-503-5p | 2.233 | up-regulation | hsa-miR-197-5p | 2.267 | down-regulation |
| hsa-miR-4269 | 2.220 | up-regulation | hsa-miR-4505 | 2.262 | down-regulation |
| hsa-miR-127-3p | 2.214 | up-regulation | hsa-miR-6075 | 2.253 | down-regulation |
| hsa-miR-4429 | 2.208 | up-regulation | hsa-miR-6756-5p | 2.236 | down-regulation |
| hsa-miR-1273g-3p | 2.206 | up-regulation | hsa-miR-4492 | 2.198 | down-regulation |
| hsa-miR-106a-5p | 2.197 | up-regulation | hsa-miR-328-5p | 2.171 | down-regulation |
| hsa-miR-27b-3p | 2.158 | up-regulation | hsa-miR-6802-5p | 2.168 | down-regulation |
| hsa-miR-125b-1-3p | 2.154 | up-regulation | hsa-miR-6869-5p | 2.160 | down-regulation |
| hsa-miR-195-5p | 2.148 | up-regulation | hsa-miR-4758-5p | 2.131 | down-regulation |
| hsa-miR-151a-5p | 2.117 | up-regulation | hsa-miR-504-3p | 2.121 | down-regulation |
| hsa-miR-30a-3p | 2.115 | up-regulation | hsa-miR-3937 | 2.116 | down-regulation |
| hsa-miR-106b-3p | 2.092 | up-regulation | hsa-miR-5572 | 2.092 | down-regulation |
| hsa-miR-532-5p | 2.083 | up-regulation | hsa-miR-3620-5p | 2.038 | down-regulation |
| hsa-miR-660-5p | 2.080 | up-regulation | hsa-miR-3124-5p | 2.028 | down-regulation |
| hsa-miR-138-5p | 2.044 | up-regulation | hsa-miR-3141 | 2.026 | down-regulation |
| hsa-miR-20a-5p | 2.043 | up-regulation | hsa-miR-4485 | 2.023 | down-regulation |
| hsa-miR-185-5p | 2.031 | up-regulation | hsa-miR-4649-5p | 2.002 | down-regulation |
| hsa-miR-152-3p | 2.017 | up-regulation | | | |

### <Experimental Example 3> in vitro anti-inflammatory effects of MSC-NVs

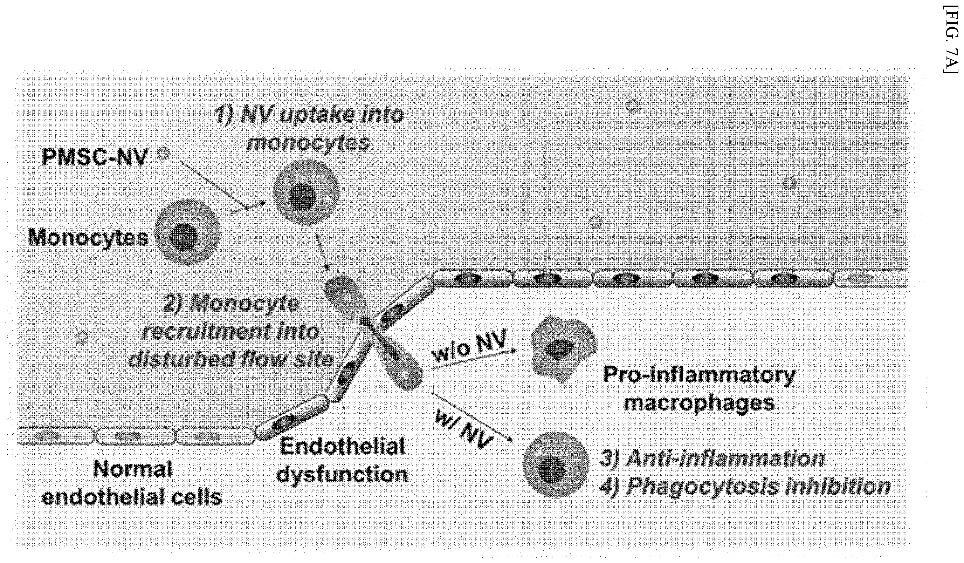

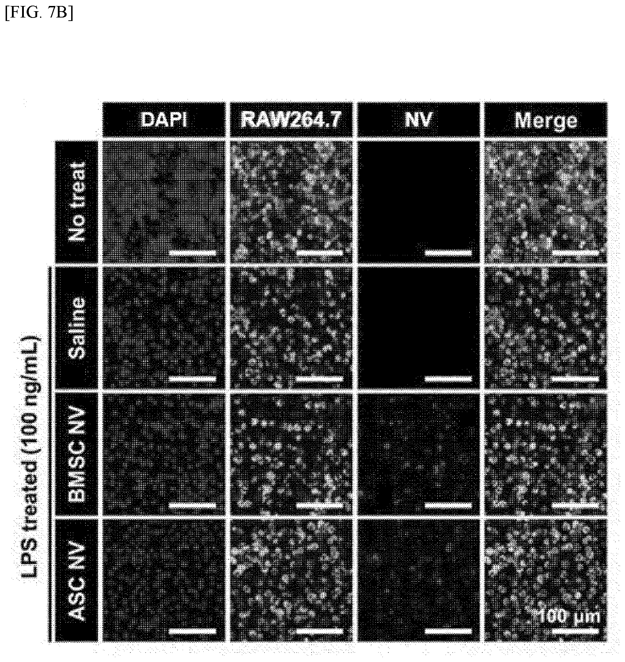

It is well known that MSCs and MSC-derived intracellular contents exhibit a potent anti-inflammatory effect. Accordingly, the anti-inflammatory effects of BMSC-NVs and ASC-NVs were examined before PREY transfection (FIG. 7A). Murine monocytes/macrophages (RAW264.7 cells) were treated with MSC-NVs to inhibit the inflammatory responses thereof. The effective cellular uptake of MSC-NVs by the activated macrophages was confirmed by visualizing the cells and MSC-NVs with DiO and DiI, respectively (FIG. 7B). Similar uptake of both ASC-NV and BMSC-NV by the activated macrophages was shown.

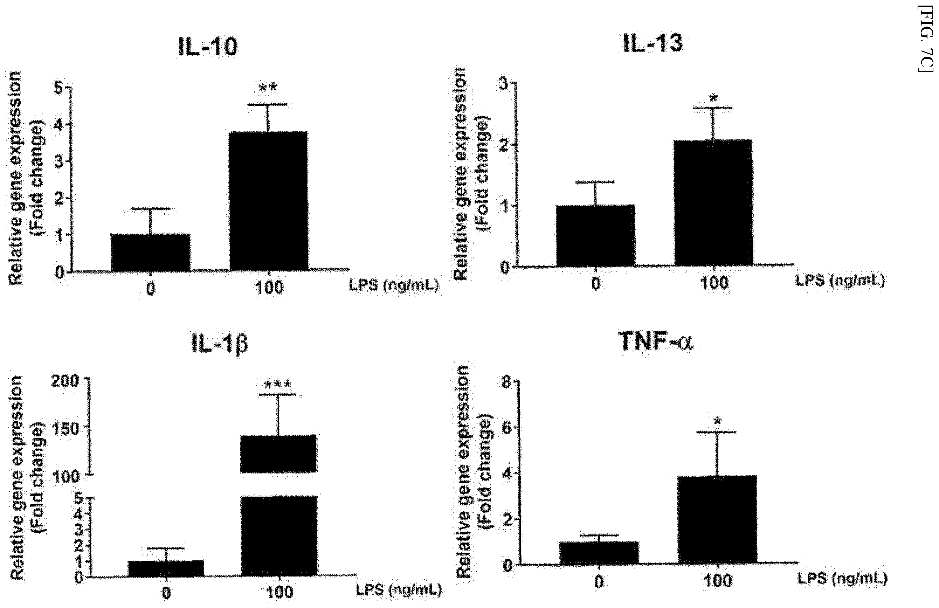

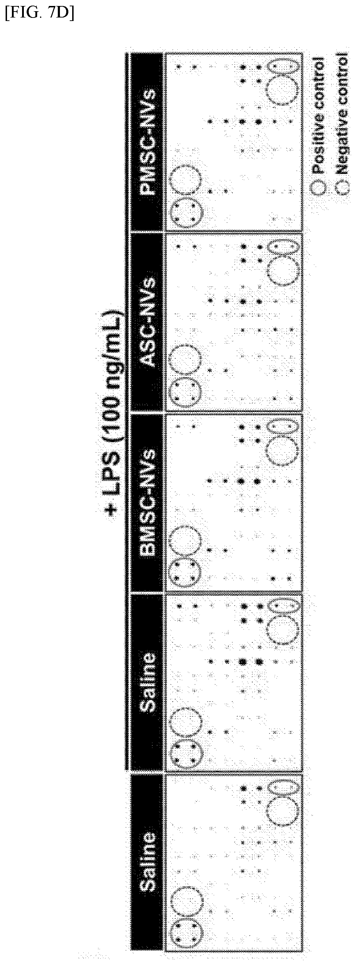

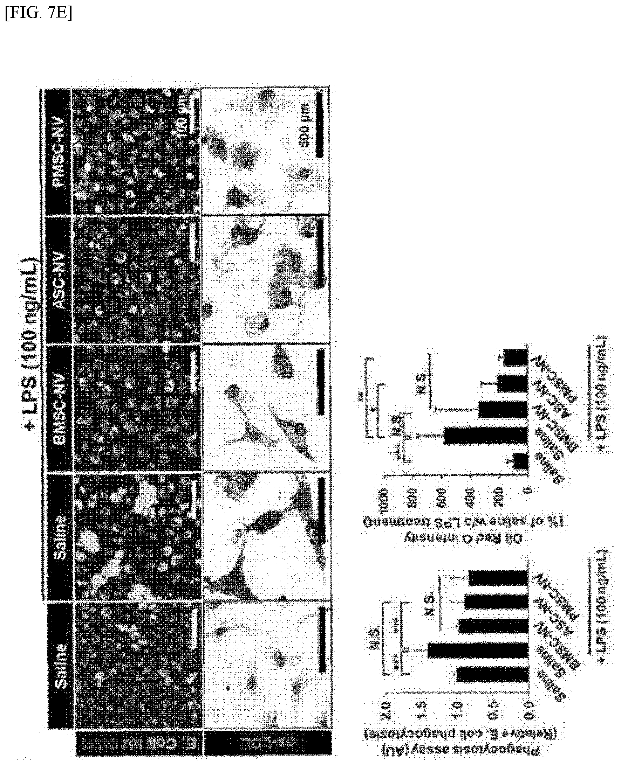

The anti-inflammatory activity of BMSC-NVs, ASC-NVs, and PMSC-NVs was analyzed by qRT-PCR (FIG. 7C), dot blot cytokine array (FIG. 7D), and phagocytosis array (FIG. 7E). The gene expression of anti-inflammatory interleukin-10 (IL-10) and IL-13 was upregulated, but that of pro-inflammatory markers (IL-1β and TNF-α) was not, indicating that the anti-inflammatory potential of macrophages by MSC-NVs was maintained even after extrusion from MSCs. These results were also supported by analysis showing an anti-inflammatory effects by cytokines secreted from conditioned media (FIG. 7D) and decreased phagocytic activity of bacterial components in all NV-treated groups (FIG. 7E). Oil red O staining for detecting the uptake level of oxidized LDL (FIG. 7E, lower panel) confirmed the reduction in oxidized LDL uptake in all MSC-NVs except BMSC-NV, supporting the inhibitory effects of ASC-NV and PMSC-NV on foam cells. The foam cell formation of macrophages caused by the uptake of oxidized LDL is a critical part in atherosclerosis development since phenotypic changes and internal growth of VSMCs are induced. These results show that MSC-NVs can prevent the atherosclerosis process.

### <Experimental Example 4> In vitro pro-EC recovery effects of MSC-NV

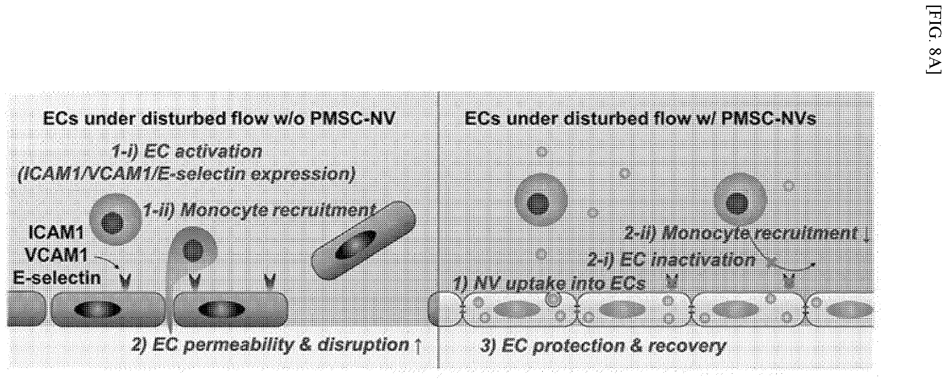

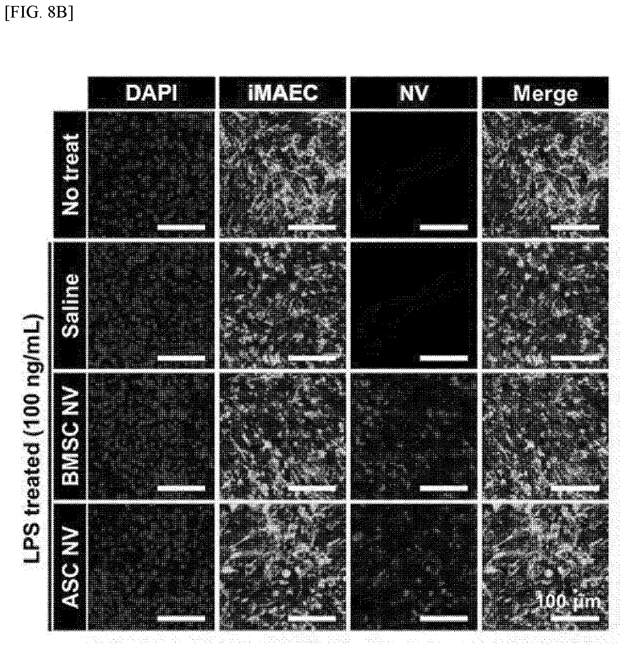

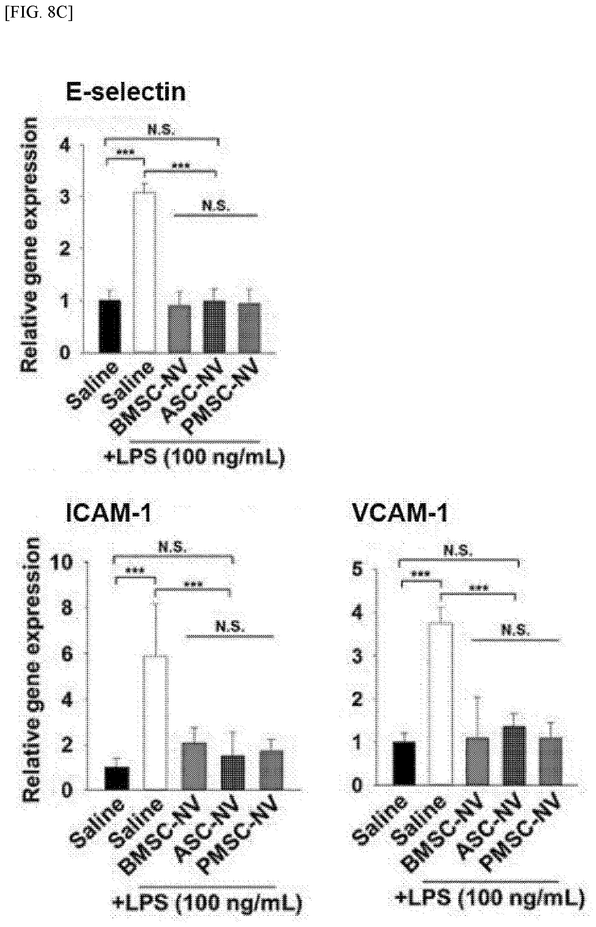

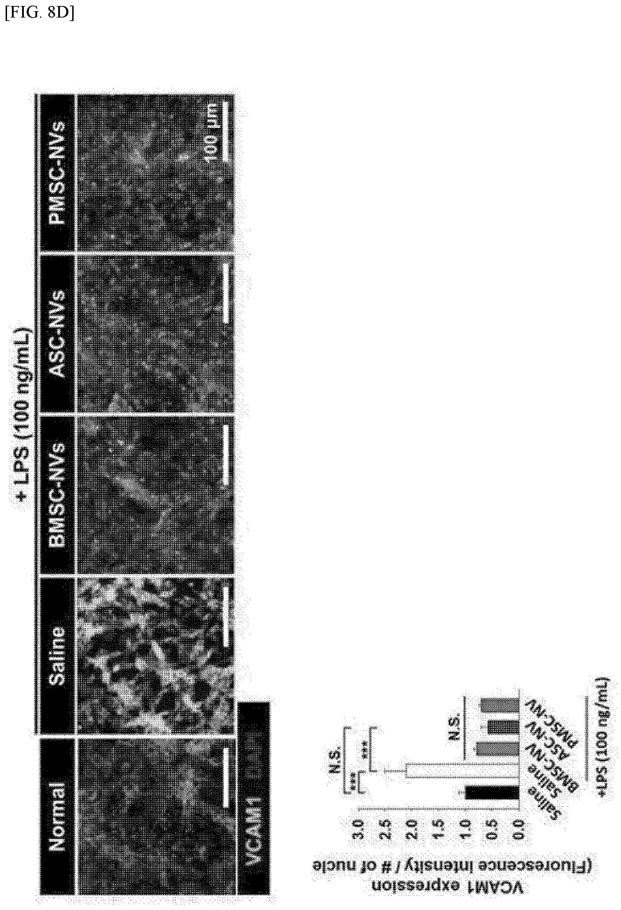

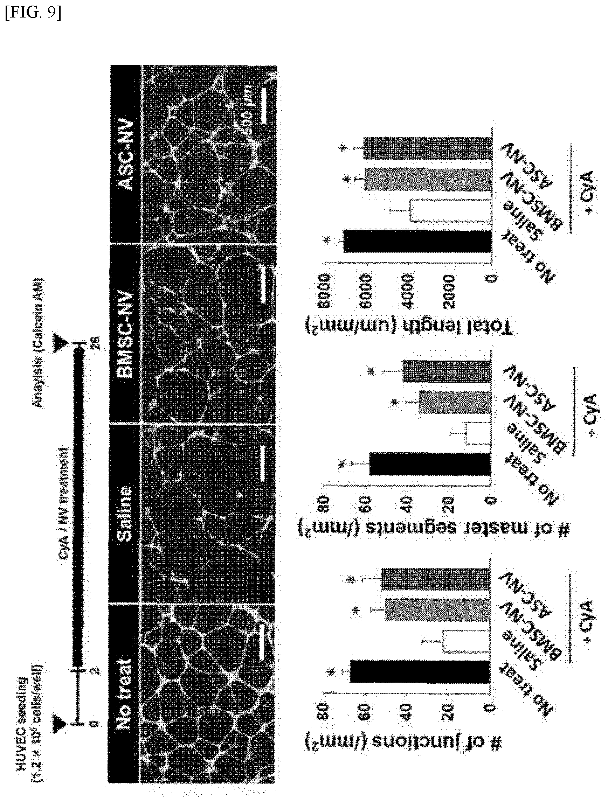

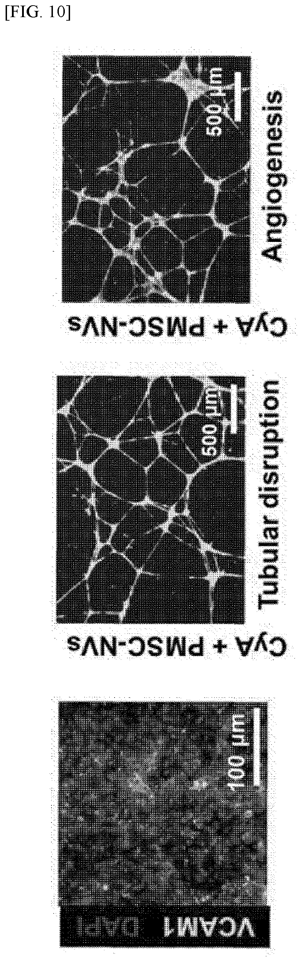

The EC-recovery effects of MSC-NV treatment was examined by activating the pro-inflammatory dysfunction of immortalized murine aortic endothelial cells (iMAEC) by LPS treatments (FIG. 8A). The effective internalization of both ASC-NVs and BMSC-NVs into activated iMAECs was visualized by labeling with DiO and DiI, respectively (FIG. 8B). There was no visible difference in NV uptake between ASC-NVs and BMSC-NVs. ECs dominantly express E-selectin, ICAM-1, and VCAM-1, and they recruit inflammatory cells upon dysfunction activation. Accordingly, when the gene expression of these markers in iMAECs is detected by qRT-PCR (FIG. 8C), both were upregulated by LPS treatments, but the gene expression significantly downregulated by treatments with both MSC-NV types or even without treatment, indicating the effective induction of EC dysfunction by 100 ng/mL of LPS as well as the promising EC-recovery effects of both MSC-NV types. This result was also supported by VCAM-1 protein expression through immunostaining since VCAM-1 is one of the initial endothelial dysfunction markers (FIG. 8D). The pro-EC repair effect was not significantly different among ASC-NVs, BMSC-NVs and PMSC-NVs (FIGS. 9 and 10), indicating that PREY transfection does not impair the therapeutic effects of MSC-NVs.

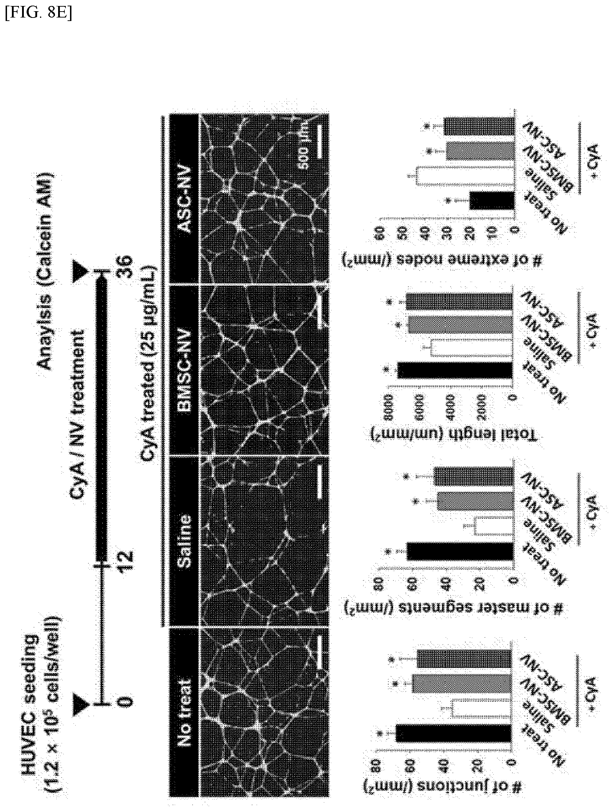

Cyclosporin A (CyA) treatments are known to disturb angiogenesis by inhibiting angiogenic EC activity. Therefore, the pro-angiogenic (FIG. 9) and pro-EC recovery (FIG. 8E) effects of MSC (BMSC vs. ASC)-NVs were examined by culturing human umbilical vein endothelial cells (HUVECs) on Matrigel and treating them with CyA and NVs 2 hours (FIG. 9) or 12 hours (FIG. 8E) after seeding. Subsequently, vasculature factors (that is, junctions, master segments, total length, and the number of extreme nodes) of HUVECs were measured. As a result, MSC-NV treatments enhanced the pro-EC recovery and pro-angiogenic effects of HUVECs in response to CyA treatments regardless of an MSC source. In addition, the consistency of the pro-EC recovery and pro-angiogenic effects of PMSC-NVs in HUVECs was confirmed upon CyA treatments at 12 hours and 2 hours after seeding, respectively (FIG. 10).

### <Experimental Example 5> In vivo targeting and anti-atherosclerotic function

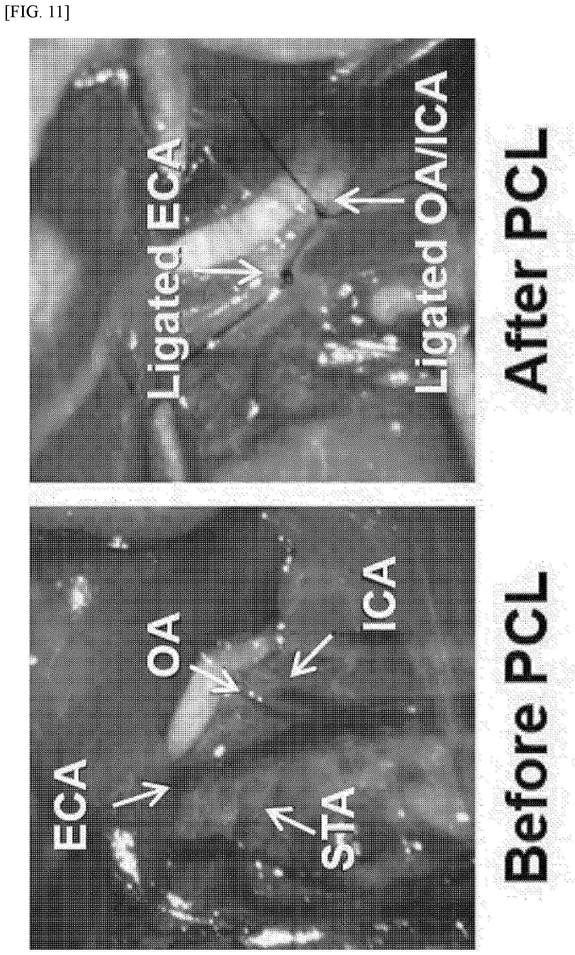

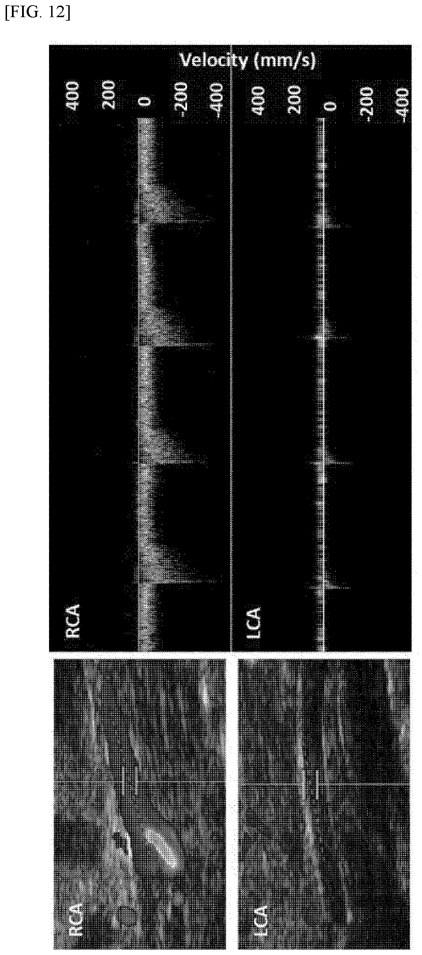

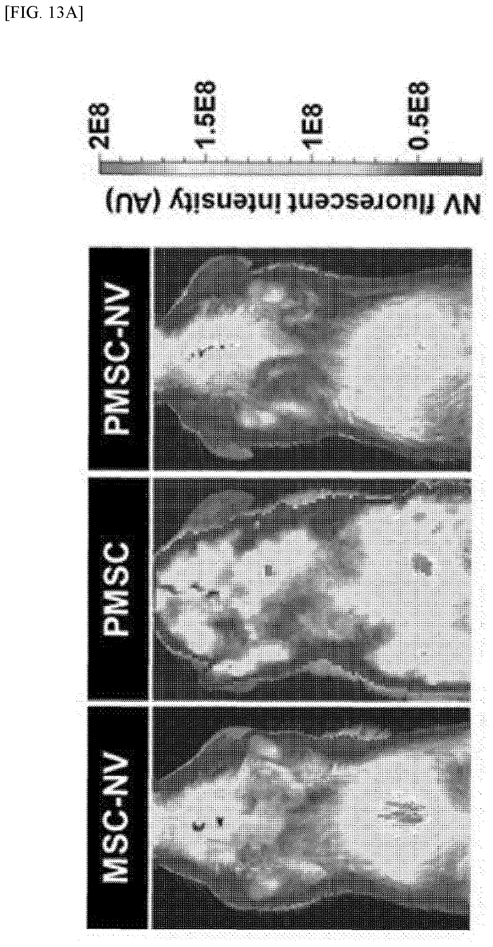

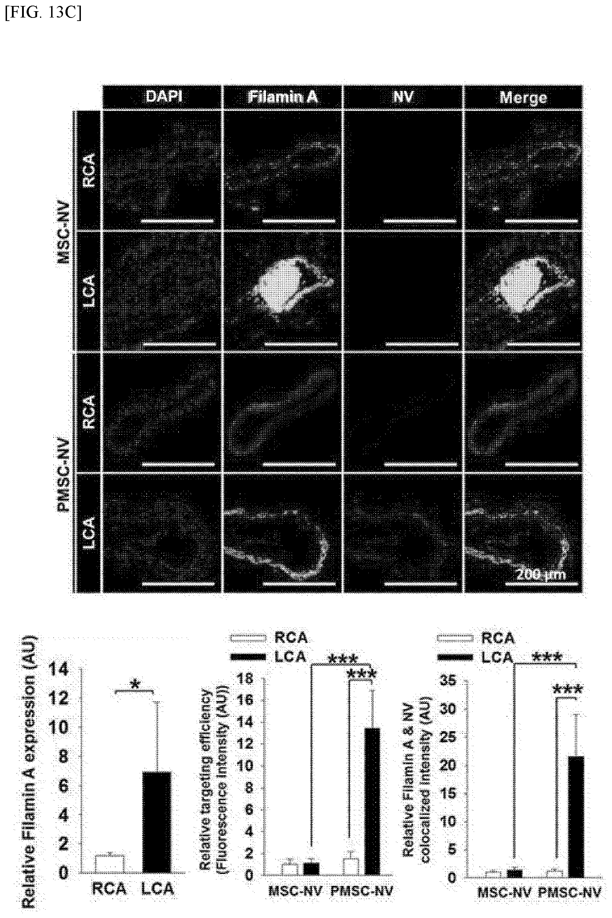

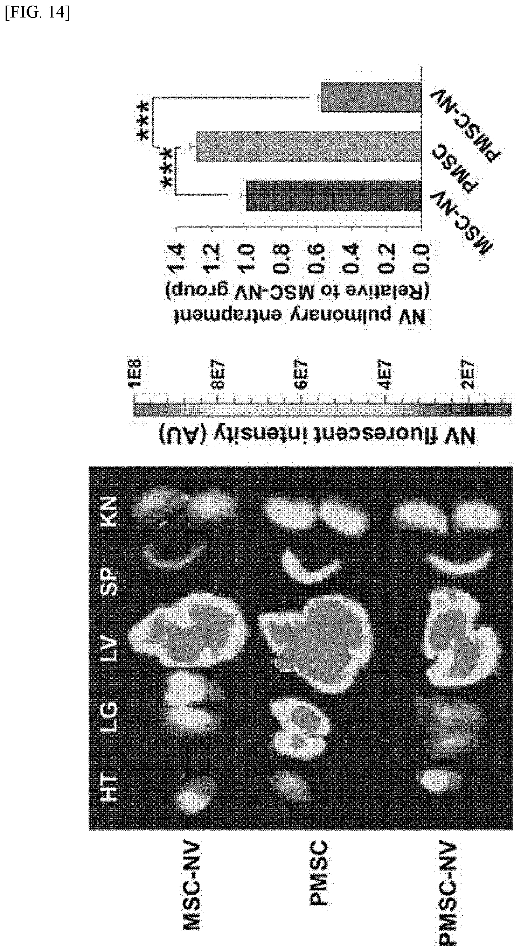

The theragnostic efficiency of PMSC-NVs was determined in a murine PCL model. As three of four left carotid artery (LCA) branches, i.e., the external carotid artery (ECA), internal carotid artery (ICA), and occipital artery (OA) were ligated (FIG. 11), the formation of a disturbed blood flow was confirmed by Doppler ultrasound imaging (FIG. 12). Subsequently, MSC-NVs, PMSC-NVs, and PMSCs were intravenously injected into the tail vein for three days after ligation. An *in vivo* imaging system (IVIS) was used to visualize the distribution of MSC-NVs, PMSC-NVs, and PMSCs in the body at 24 hours after injection (FIG. 13A-B, FIG. 14). The LCA-targeting efficiency of PMSC-NVs was higher than that of MSC-NVs or PMSCs, indicating the synergistic roles of PREY and NV to disturb the pulmonary capillary entrapment of cells and effectively target disturbed blood flow sites. Pulmonary capillary entrapment is the major obstacle for systemic cell delivery. The inventors identified filamin A as a target molecule of PREY through a previous proteomic analysis. The RCA and LCA were obtained and immunostained 24 hours after injection (FIG. 13C). Filamin A expression was significantly higher in the LCA than in RCA, confirming its overexpression at the disturbed blood flow site for PMSC recruitment. Among the test groups in which NVs were labeled with Vivotrack680, only PMSC-NVs were clearly co-internalized with filamin A.

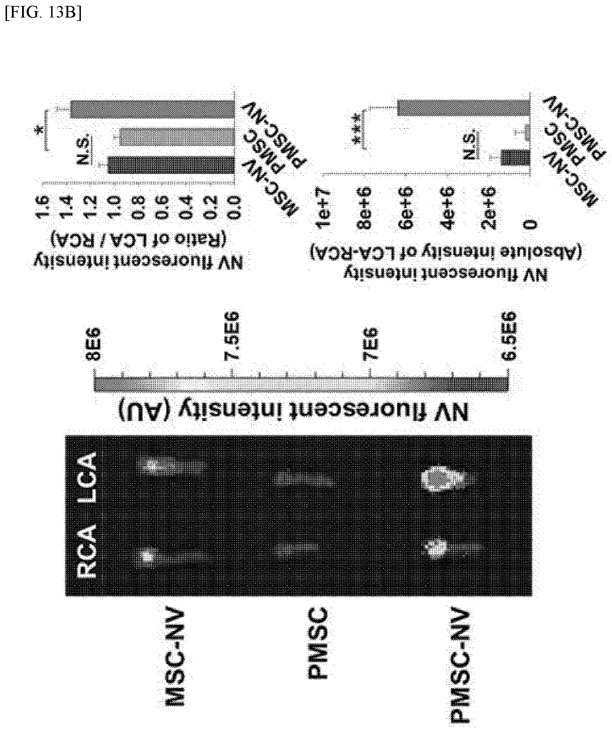

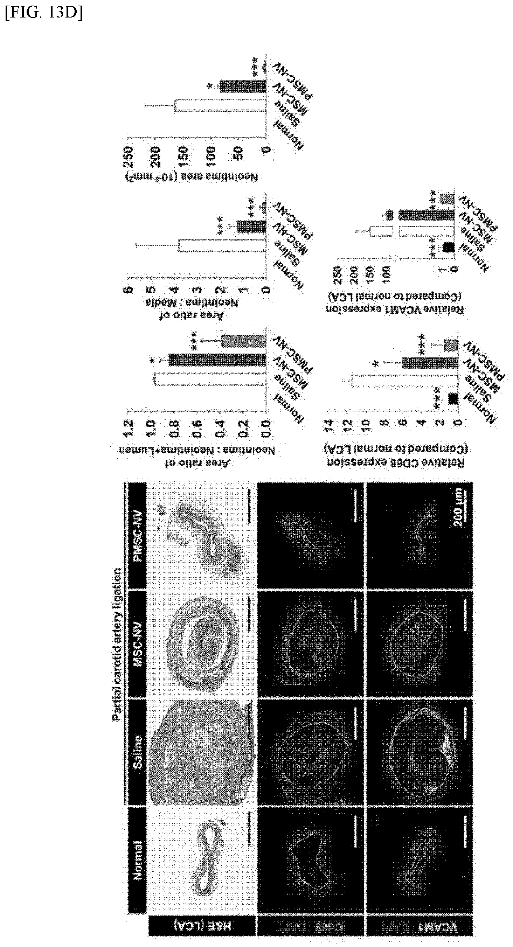

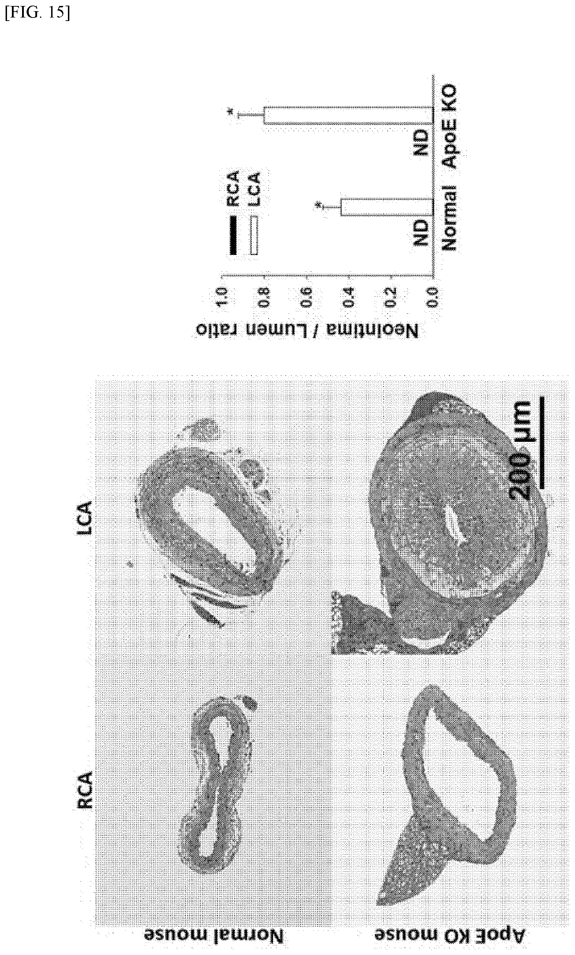

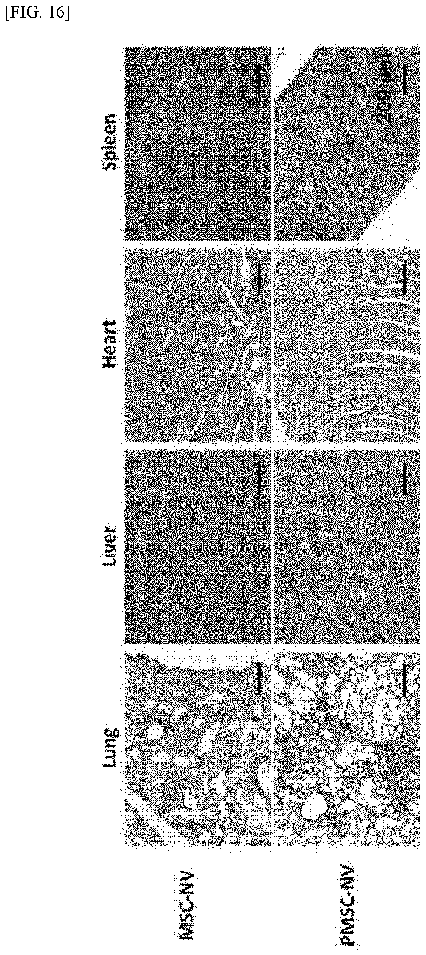

Since the anti-inflammatory and pro-EC recovery effects of MSC-NVs (FIGS.7A to E, FIGS. 8A to E, FIG. 9 and FIG. 10) and the PREY targeting efficiency (FIG. 13A to C) had been demonstrated, it was hypothesized that their combination (PMSC-NV) can exhibit a synergistic theragnostic effects that prevent early progression of atherosclerosis. ApoE KO mice that underwent PCL surgery were used for this part of this experiment. This is because this mouse strain showed more aggressive atherosclerosis development compared with the corresponding normal strain (FIG. 15). When the LCA and RCA were obtained from the ApoE KO PCL model 11 days after NV injection, PMSC-NVs maintain a similar vascular form to a normal control and effectively prevent angiogenesis, but other test groups did not show clear therapeutic effects (top row of FIG. 13D). Meanwhile, since the dose of PMSCs was not sufficient for allowing effective targeting, angiogenesis could not be effectively prevented in comparison with PMSC-NVs (FIG. 13B). Protein expression levels of CD68 (middle row of FIG. 13D) and VCAM-1 (bottom row of FIG. 13D) were determined as indications of monocyte recruitment and EC activation, respectively. When PMSC-NVs were treated, their expression level decreased to that of the control without exhibiting visible toxic effects in the heart, lungs, liver, and spleen after systemic circulation (FIG. 16). In contrast, the other test groups exhibited significantly higher expression of markers compared to those of the normal controls and PMSC-NV groups.

### <Experimental Examples 6> In vivo porcine PCL and in vitro human EC models

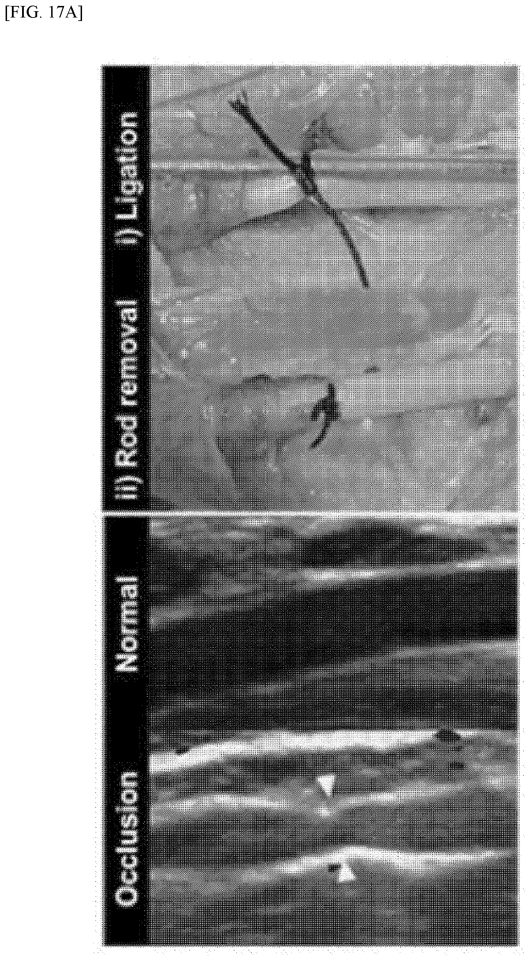

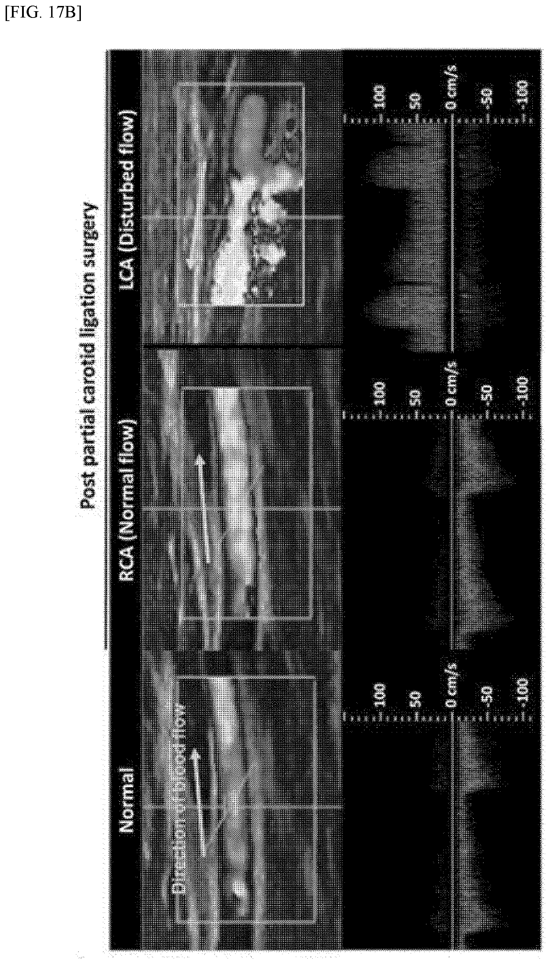

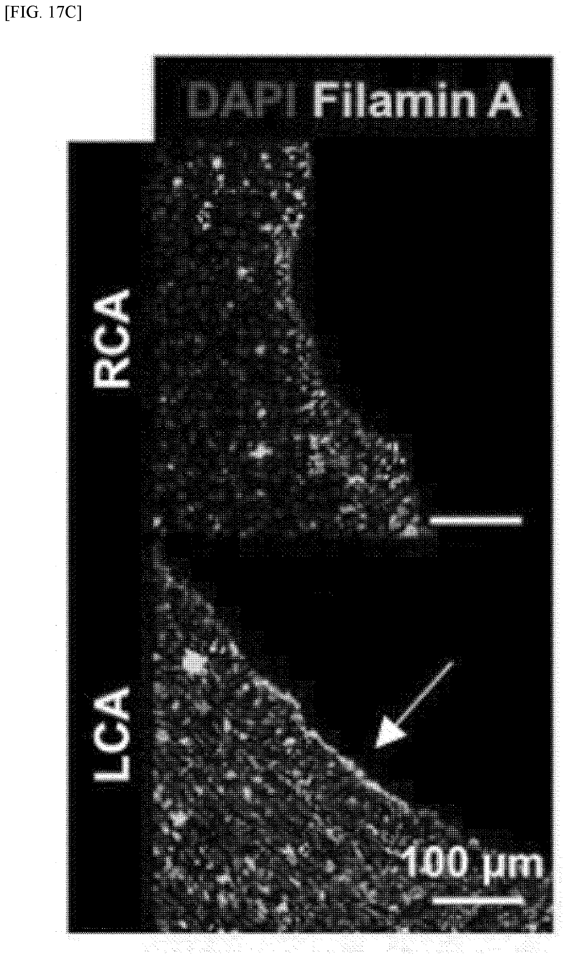

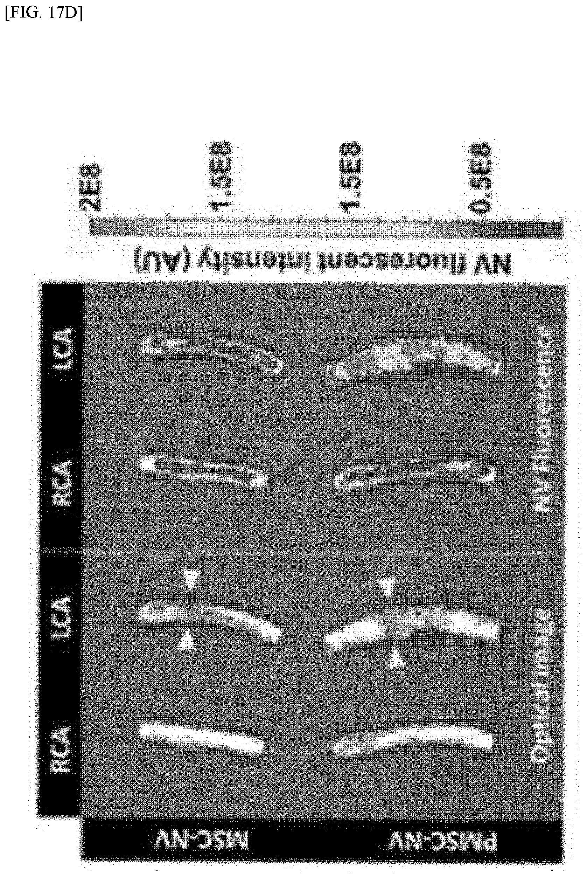

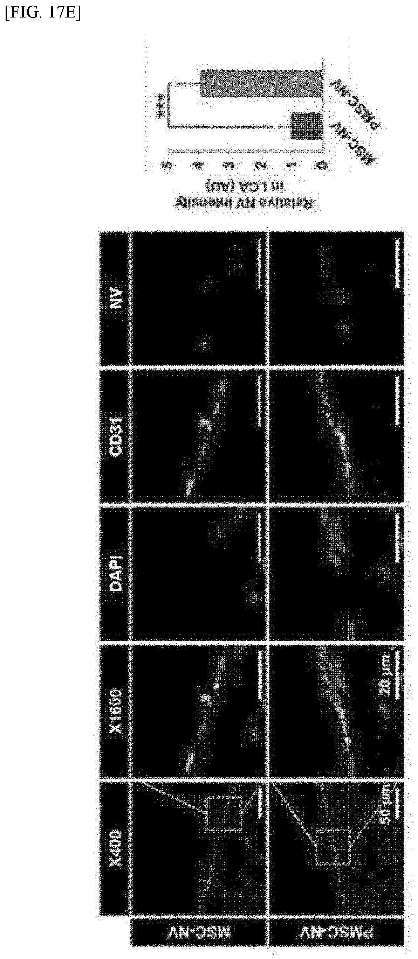

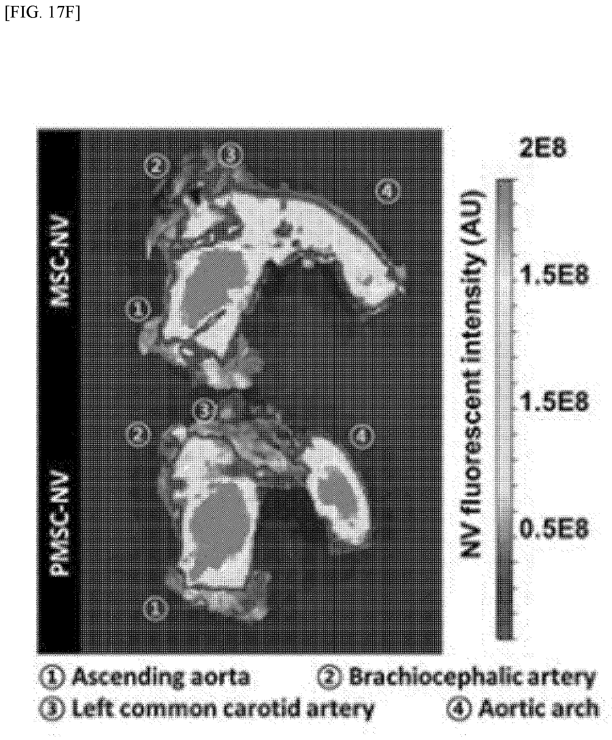

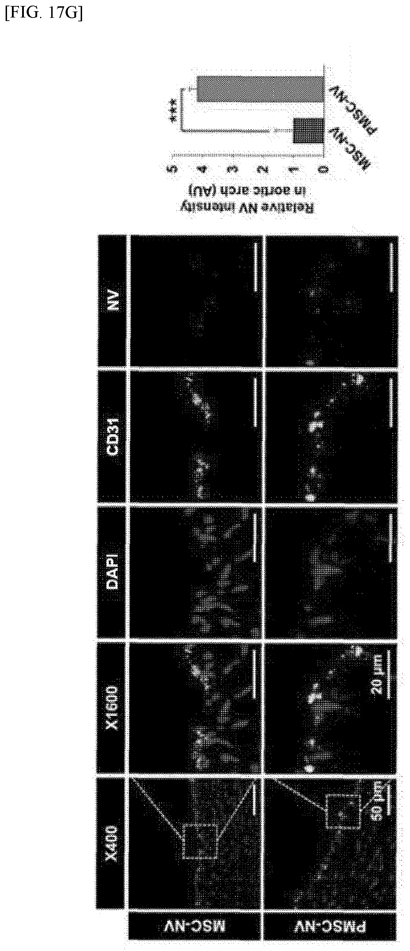

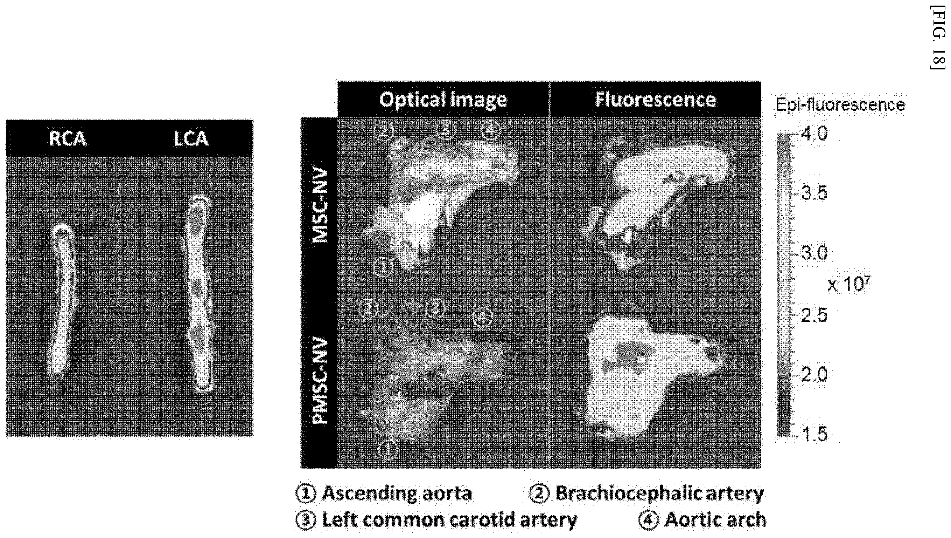

As a pre-clinical large animal model, a porcine PCL model was used to confirm the targeting efficiency of PMSC-NVs for disturbed blood flow sites according to a previously established method. Briefly, a stainless-steel rod (diameter=0.9mm) was tightly ligated to the LCA (diameter=4.5mm) and then removed (top, FIG. 17A), inducing 70 to 80% LCA occlusion (bottom, FIG. 17B). Clear formation of a disturbed blood flow at the distal part of the ligated LCA was confirmed by Doppler ultrasound imaging (FIG. 17B). In addition, filamin A was highly expressed in the endothelium of the LCA compared to the RCA (FIG. 17C). Vivotrack680-labeled MSC-NVs or PMSC-NVs were intravenously administered through the ear vein, and systemically circulated for 3 days after ligation. The RCA, LCA, and aortic arch were harvested from tissue samples of normal, induced disturbed blood flow, and naturally disturbed blood flow sites 24 hours or 21 hours after NV injection. The naturally disturbed blood flow is formed in an aortic arch due to its curvature and branching structure. IVIS images demonstrated that PMSC-NVs are highly accumulated in the distal region of the ligation point of the LCA, which is an indicator of pathogenic remodeling, but they were not observed in the RCA (FIG. 17D, left of FIG. 18). In the aortic arch samples, the IVIS fluorescence distribution of PMSC-NVs was highly correlated with the disturbed blood flow sites compared with MSC-NVs, indicating the effective targeting function of PREY (FIG. 17F, right of FIG. 18), which was supported by the greater co-existence of CD31 with PMSC-NVs than MSC-NVs (FIGS. 17E and 17G).

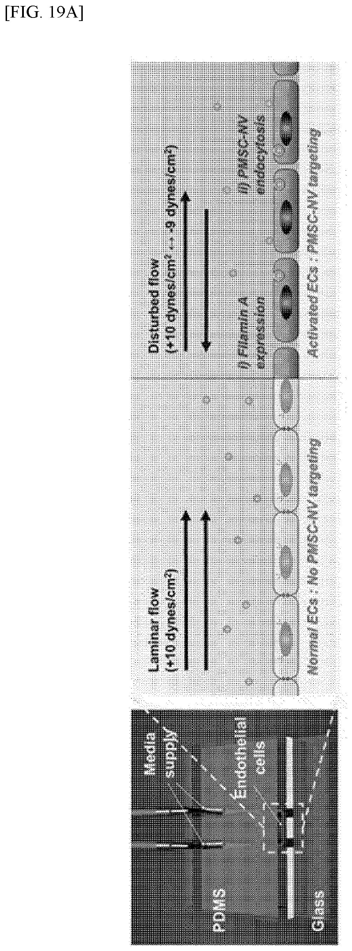

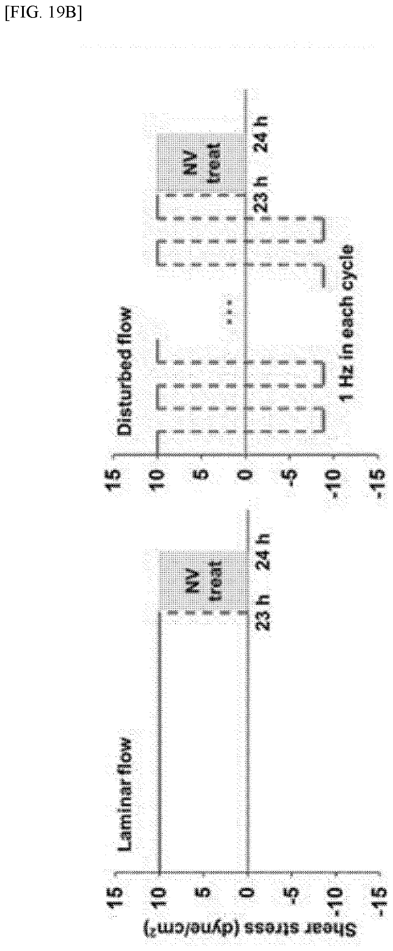

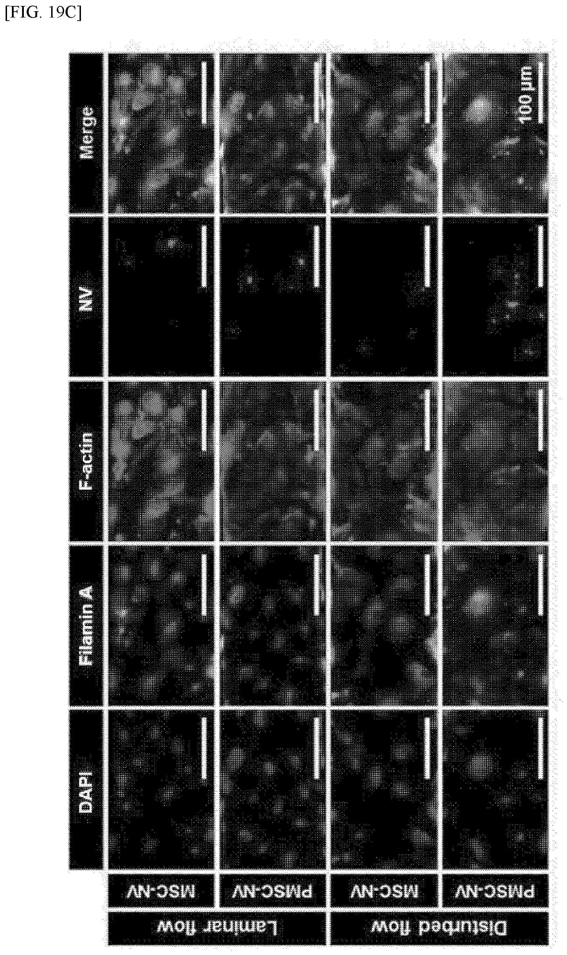

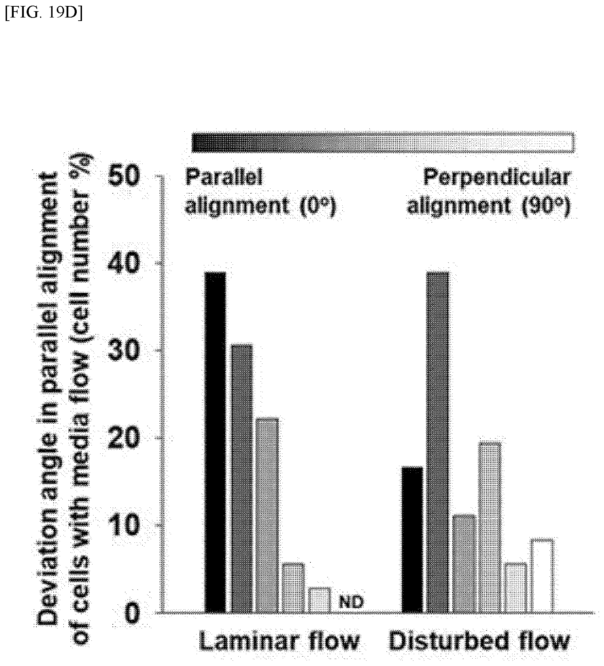

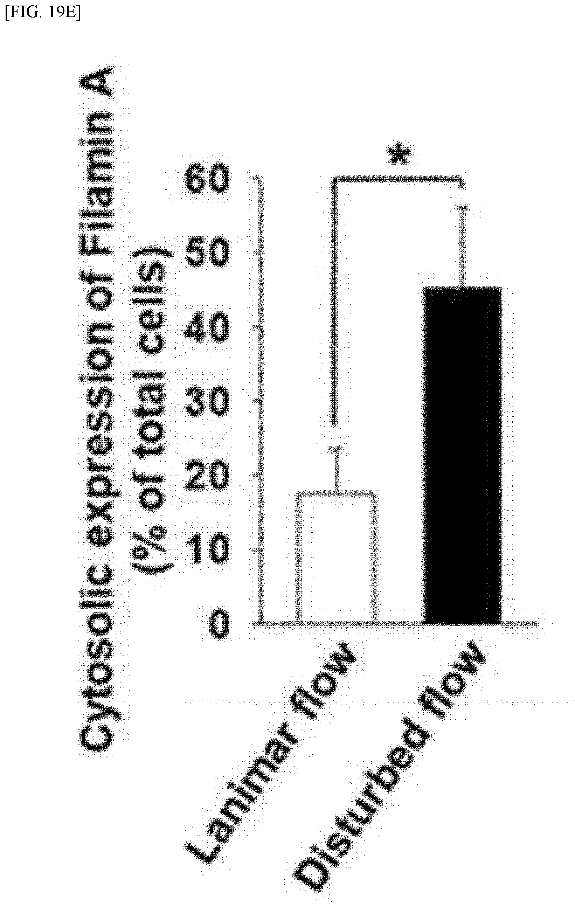

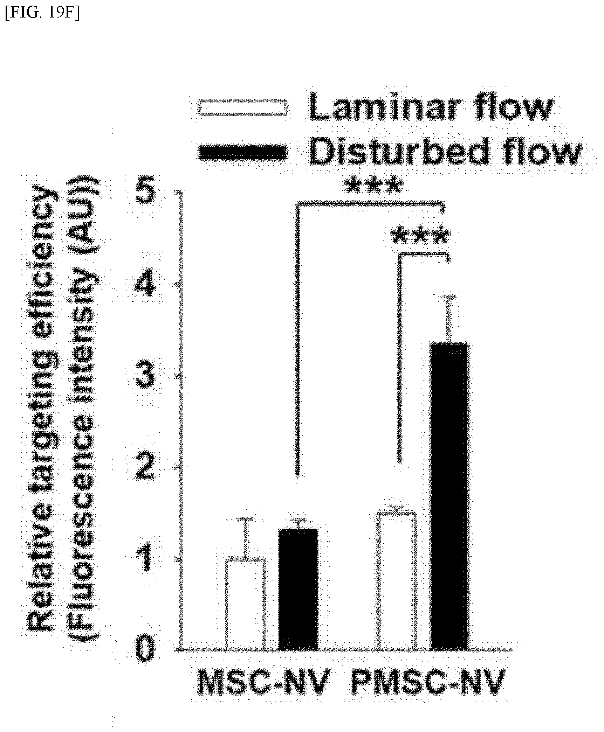

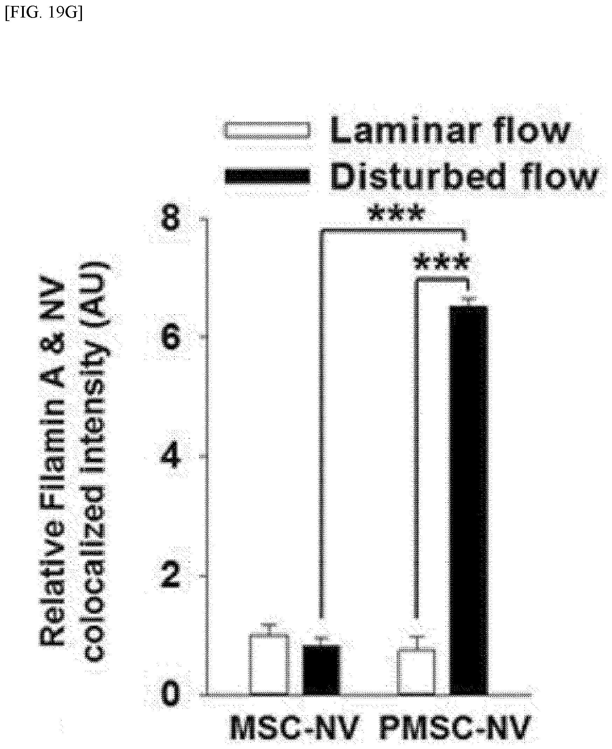

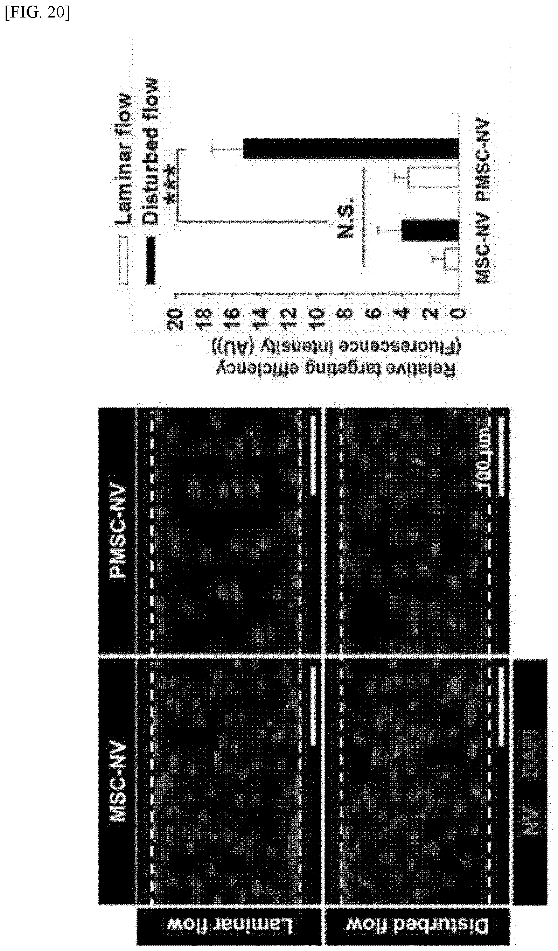

Finally, under a disturbed blood flow, the efficiency of PMSC-NV targeting hCAECs (FIGS. 19A to G) and human aortic ECs (hAECs; FIG. 20) were detected using an *in vitro* microfluidic model. In the microfluidic model, the EC monolayer attached to a collagen-coated glass surface was exposed to a medium flow generated by a pumping system, which successfully mimics blood flow under a healthy or atherosclerotic condition. In addition, the microfluidic system enables precise observation of target sites as well as the targeting efficiency of PMSC-NVs, which is difficult to achieve in an *in vivo* experiment. To mimic healthy and atherosclerotic conditions of blood vessels, ECs were exposed to normal (10 dyne/cm²) or disturbed blood flow (10 dyne/cm² and -9 dyne/cm² with 1 Hz pulse) (FIGS. 19A to C) before MSC-NV treatments. It was confirmed that ECs exposed to normal flow were aligned to the flow direction, whereas ECs exposed to disturbed blood flow were not (FIG. 19D). In addition, filamin A expression increased under a disturbed blood flow (FIG. 19E), whereas F-actin expression was similar between ECs exposed to normal flow and disturbed blood flow (FIG. 19F). More importantly, it was revealed that, in the microfluidic model, the signals of PMSC-NVs increased under a disturbed blood flow condition (FIG. 20), compared with the normal flow condition, due to high coexistence with filamin A, proving the theragnostic potential of PMSC-NVs in clinical interpretation.

As above, as specific parts of the present invention have been described in detail, although it is clear to those skilled in the art that this specific technique is merely a preferred embodiment, the scope of the specification is not limited thereto.

### [Industrial applicability]

Since stem cell-derived nanovesicles using a peptide capable of targeting a disturbed blood flow site according to the present invention provide potent anti-inflammatory and pre-endothelial recovery effects similar to mesenchymal stem cells as an anti-atherosclerosis theragnostic platform, they are expected to be effectively used in related medical industry as a novel theragnostic agent for preventing and treating atherosclerosis.

## Claims

1. Stem cell-derived nanovesicles displaying a disturbed blood flow site-targeting peptide on the surface thereof.

2. The nanovesicles of claim 1, wherein the disturbed blood flow site-targeting peptide is selected from the group consisting of SEQ ID NOs: 1 to 5.

3. The nanovesicles of claim 1, wherein the nanovesicles are derived from stem cells transfected with a vector into which coding sequences of signal peptide, disturbed blood flow site-targeting peptide, and transmembrane protein are sequentially inserted.

4. The nanovesicles of claim 3, wherein the transmembrane protein is one or more selected from the group consisting of CD86, CD105, CD271, CD34, and CD22.

5. The nanovesicles of claim 1, wherein the stem cells are any one of stem cells derived from one or more of tissue selected from the group consisting of bone marrow, the umbilical cord, umbilical cord blood, the placenta, blood, skin, adipose tissue, nervous tissue, the liver, the pancreatic duct, muscle, and the amniotic membrane; mesenchymal stem cells; embryonic stem cells; and induced pluripotent stem cells.

6. A method of preparing the nanovesicles of claim 1, comprising the step of obtaining nanovesicles displaying a disturbed blood flow site-targeting peptide on the surface thereof from stem cells transfected with a vector into which coding sequences of signal peptide, disturbed blood flow site-targeting peptide, and transmembrane protein are sequentially inserted.

7. A composition for diagnosing atherosclerosis, comprising the nanovesicles of claim 1.

8. A composition for preventing or treating atherosclerosis, comprising the nanovesicles of claim 1.

9. A method of diagnosing atherosclerosis, comprising the step of administering the nanovesicles of claim 1 into a subject.

10. A method of preventing or treating atherosclerosis, comprising the step of administering the nanovesicles of claim 1 into a subject.

11. A use of the nanovesicles of claim 1 for preventing or treating atherosclerosis.


<110>    NUMAIS CO., LTD.

<120>    Method for diagnosing and treating atherosclerosis by using stem
         cell nanovesicle targeting disturbed flow sites

<130>    PCT2020-039

<150>    KR 10-2019-0107218
<151>    2019-08-30

<160>    43

<170>    KoPatentIn 3.0

<210>    1
<211>    12
<212>    PRT
<213>    Artificial Sequence

<220>
<223>    PREY peptide


<400>    1
Gly Ser Pro Arg Glu Tyr Thr Ser Tyr Met Pro His
  1               5                   10


<210>    2
<211>    11
<212>    PRT
<213>    Artificial Sequence

<220>
<223>    Myoferlin peptide


<400>    2
Ser Pro Arg Glu Tyr Thr Ser Tyr Met Pro His
  1               5                   10


<210>    3
<211>    12
<212>    PRT
<213>    Artificial Sequence

<220>
<223>    Eyes absent homolog 1 isoform 2 peptide


<400>    3
Ser Leu Ser Ser Tyr Asn Gly Ser Ala Leu Ala Ser
  1               5                   10


<210>    4
<211>    10
<212>    PRT
<213>    Artificial Sequence

<220>
<223>    Zinc finger protein, partial  peptide

<400>    4
Ala Cys Asn Thr Gly Ser Pro Tyr Glu Cys
1               5                   10

<210>    5
<211>    10
<212>    PRT
<213>    Artificial Sequence

<220>
<223>    Calsyntenin 1, isoform CRA_b  peptide

<400>    5
Ala Cys Thr Pro Ser Phe Ser Lys Ile Cys
1               5                   10

<210>    6
<211>    22
<212>    DNA
<213>    Artificial Sequence

<220>
<223>    pCMV Backbone_F

<400>    6
taactcgagc atgcatctag ag                                                22

<210>    7
<211>    20
<212>    DNA
<213>    Artificial Sequence

<220>
<223>    pCMV Backbone_R

<400>    7
ggtggcaagc ttgggtctcc                                                   20

<210>    8
<211>    50
<212>    DNA
<213>    Artificial Sequence

<220>
<223>    mEGFP_F

<400>    8
gaagcggagg aggaggaagc ggaggaggag gtagcgtgag caagggcgag                  50

<210>    9
<211>    52
<212>    DNA

<213>    Artificial Sequence

<220>
<223>    mEGFP_R


<400>    9
atagggccct ctagatgcat gctcgagtta ttacttgtac agctcgtcca tg          52


<210>    10
<211>    50
<212>    DNA
<213>    Artificial Sequence

<220>
<223>    CD86 Signal_F


<400>    10
ctcactatag ggagacccaa gcttgccacc atggatcccc agtgcactat          50


<210>    11
<211>    56
<212>    DNA
<213>    Artificial Sequence

<220>
<223>    CD86_Signal_PREY_R


<400>    11
gtgaggcata taggaggtgt actcgcgggg actgccagct tgaatcttca gaggag          56


<210>    12
<211>    47
<212>    DNA
<213>    Artificial Sequence

<220>
<223>    NGFR Signal_F


<400>    12
ctcactatag ggagacccaa gcttgccacc atggacgggc cgcgcct          47


<210>    13
<211>    50
<212>    DNA
<213>    Artificial Sequence

<220>
<223>    NGFR Signal_R


<400>    13
catataggag gtgtactcgc ggggactgcc ggcacctcca agggacaccc          50


<210>    14

<211>    50
<212>    DNA
<213>    Artificial Sequence

<220>
<223>    CD105 Signal_F


<400>    14
ctcactatag ggagacccaa gcttgccacc atggaccgcg gcacgctccc                50


<210>    15
<211>    50
<212>    DNA
<213>    Artificial Sequence

<220>
<223>    CD105 Signal_R


<400>    15
catataggag gtgtactcgc ggggactgcc tgcaagactt gtggggctga                50


<210>    16
<211>    56
<212>    DNA
<213>    Artificial Sequence

<220>
<223>    V5 tag_PREY_F


<400>    16
ggcagtcccc gcgagtacac ctcctatatg cctcacggca agcccatccc caaccc          56


<210>    17
<211>    50
<212>    DNA
<213>    Artificial Sequence

<220>
<223>    V5 tag_CD86_R


<400>    17
gcatggcagg tctgcagtct cattgaaata ggtgctgtcc aggcccagca                50


<210>    18
<211>    42
<212>    DNA
<213>    Artificial Sequence

<220>
<223>    V5 Full AS


<400>    18
ggtgctgtcc aggcccagca gggggttggg gatgggcttg cc                        42

<210>    19
<211>    23
<212>    DNA
<213>    Artificial Sequence

<220>
<223>    CD86 MP_F


<400>    19
tatttcaatg agactgcaga cct                                                23


<210>    20
<211>    57
<212>    DNA
<213>    Artificial Sequence

<220>
<223>    CD86 MP_Linker_R


<400>    20
tccgcttcct cctcctccgc ttccacctcc tccaaaacat gtatcacttt tgtcgca          57


<210>    21
<211>    49
<212>    DNA
<213>    Artificial Sequence

<220>
<223>    V5_LNGFR_F


<400>    21
cccaaccccc tgctgggcct ggacagcacc aaggaggcat gccccacag                    49


<210>    22
<211>    55
<212>    DNA
<213>    Artificial Sequence

<220>
<223>    LNGFR_Linker_R


<400>    22
ctccgcttcc tcctcctccg cttccacctc ctcctcgata agcttggctg caggt            55


<210>    23
<211>    50
<212>    DNA
<213>    Artificial Sequence

<220>
<223>    V5-CD105_F


<400>    23

cccaaccccc tgctgggcct ggacagcacc gaaacagtcc attgtgacct 50

<210> 24
<211> 54
<212> DNA
<213> Artificial Sequence

<220>
<223> CD105-GS_R

<400> 24
ctccgcttcc tcctcctccg cttccacctc ctcctgccat gctgctggtg gagc 54

<210> 25
<211> 1701
<212> DNA
<213> Artificial Sequence

<220>
<223> PREY-CD271-EGFP plasmid

<400> 25
atggacgggc cgcgcctgct gctgttgctg cttctggggg tgtcccttgg aggtgccggc 60

agtccccgcg agtacacctc ctatatgcct cacggcaagc ccatccccaa ccccctgctg 120

ggcctggaca gcaccaagga ggcatgcccc acaggcctgt acacacacag cggtgagtgc 180

tgcaaagcct gcaacctggg cgagggtgtg gcccagcctt gtggagccaa ccagaccgtg 240

tgtgagccct gcctggacag cgtgacgttc tccgacgtgg tgagcgcgac cgagccgtgc 300

aagccgtgca ccgagtgcgt ggggctccag agcatgtcgg cgccgtgcgt ggaggccgac 360

gacgccgtgt gccgctgcgc ctacggctac taccaggatg agacgactgg gcgctgcgag 420

gcgtgccgcg tgtgcgaggc gggctcgggc ctcgtgttct cctgccagga caagcagaac 480

accgtgtgcg aggagtgccc cgacggcacg tattccgacg aggccaacca cgtggacccg 540

tgcctgccct gcaccgtgtg cgaggacacc gagcgccagc tccgcgagtg cacacgctgg 600

gccgacgccg agtgcgagga gatccctggc cgttggatta cacggtccac acccccagag 660

ggctcggaca gcacagcccc cagcacccag gagcctgagg cacctccaga acaagacctc 720

atagccagca cggtggcagg tgtggtgacc acagtgatgg gcagctccca gcccgtggtg 780

acccgaggca ccaccgacaa cctcatccct gtctattgct ccatcctggc tgctgtggtt 840

gtgggccttg tggcctacat agccttcaag aggtggaaca gtcatcgata tcctcgaggt 900

caccgcggtc tagagtcgac ctgcagccaa gcttatcgag gaggaggtgg aagcggagga 960

ggaggaagcg gaggaggagg tagcgtgagc aagggcgagg agctgttcac cggggtggtg 1020

cccatcctgg tcgagctgga cggcgacgta aacggccaca agttcagcgt gtccggcgag 1080

ggcgagggcg atgccaccta cggcaagctg accctgaagt tcatctgcac caccggcaag 1140

ctgcccgtgc cctggcccac cctcgtgacc accctgacct acggcgtgca gtgcttcagc 1200

cgctaccccg accacatgaa gcagcacgac ttcttcaagt ccgccatgcc cgaaggctac 1260

gtccaggagc gcaccatctt cttcaaggac gacggcaact acaagacccg cgccgaggtg 1320

aagttcgagg gcgacaccct ggtgaaccgc atcgagctga agggcatcga cttcaaggag 1380

gacggcaaca tcctggggca caagctggag tacaactaca acagccacaa cgtctatatc 1440

atggccgaca agcagaagaa cggcatcaag gtgaacttca agatccgcca caacatcgag 1500

gacggcagcg tgcagctcgc cgaccactac cagcagaaca cccccatcgg cgacggcccc 1560

gtgctgctgc ccgacaacca ctacctgagc acccagtccg ccctgagcaa agaccccaac 1620

gagaagcgcg atcacatggt cctgctggag ttcgtgaccg ccgccgggat cactctcggc 1680

atggacgagc tgtacaagta a 1701

<210> 26
<211> 1827
<212> DNA
<213> Artificial Sequence

<220>
<223> PREY-CD86-EGFP plasmid

<400> 26
atggatcccc agtgcactat gggactgagt aacattctct ttgtgatggc cttcctgctc 60

tctggtgctg ctcctctgaa gattcaagct ggcagtcccc gcgagtacac ctcctatatg 120

cctcacggca agcccatccc caaccccctg ctgggcctgg acagcaccta tttcaatgag 180

actgcagacc tgccatgcca atttgcaaac tctcaaaacc aaagcctgag tgagctagta 240

gtattttggc aggaccagga aaacttggtt ctgaatgagg tatacttagg caaagagaaa 300

tttgacagtg ttcattccaa gtatatgggc cgcacaagtt ttgattcgga cagttggacc 360

ctgagacttc acaatcttca gatcaaggac aagggcttgt atcaatgtat catccatcac 420

aaaaagccca caggaatgat tcgcatccac cagatgaatt ctgaactgtc agtgcttgct 480

aacttcagtc aacctgaaat agtaccaatt tctaatataa cagaaaatgt gtacataaat 540

ttgacctgct catctataca cggttaccca gaacctaaga agatgagtgt tttgctaaga 600

accaagaatt caactatcga gtatgatggt attatgcaga aatctcaaga taatgtcaca 660

gaactgtacg acgtttccat cagcttgtct gtttcattcc ctgatgttac gagcaatatg 720

accatcttct gtattctgga aactgacaag acgcggcttt tatcttcacc tttctctata 780

gagcttgagg accctcagcc tcccccagac cacattcctt ggattacagc tgtacttcca 840

acagttatta tatgtgtgat ggttttctgt ctaattctat ggaaatggaa gaagaagaag 900

cggcctcgca actcttataa atgtggaacc aacacaatgg agagggaaga gagtgaacag 960

accaagaaaa gagaaaaaat ccatatacct gaaagatctg atgaagccca gcgtgttttt 1020

aaaagttcga agacatcttc atgcgacaaa agtgatacat gttttggagg aggtggaagc 1080

ggaggaggag gaagcggagg aggaggtagc gtgagcaagg gcgaggagct gttcaccggg 1140

gtggtgccca tcctggtcga gctggacggc gacgtaaacg gccacaagtt cagcgtgtcc 1200

ggcgagggcg agggcgatgc cacctacggc aagctgaccc tgaagttcat ctgcaccacc 1260

ggcaagctgc ccgtgccctg gcccaccctc gtgaccaccc tgacctacgg cgtgcagtgc 1320

ttcagccgct accccgacca catgaagcag cacgacttct tcaagtccgc catgcccgaa 1380

ggctacgtcc aggagcgcac catcttcttc aaggacgacg gcaactacaa gacccgcgcc 1440

gaggtgaagt tcgagggcga caccctggtg aaccgcatcg agctgaaggg catcgacttc 1500

aaggaggacg gcaacatcct ggggcacaag ctggagtaca actacaacag ccacaacgtc 1560

tatatcatgg ccgacaagca gaagaacggc atcaaggtga acttcaagat ccgccacaac 1620

atcgaggacg gcagcgtgca gctcgccgac cactaccagc agaacacccc catcggcgac 1680

ggccccgtgc tgctgcccga caaccactac ctgagcaccc agtccgccct gagcaaagac 1740

cccaacgaga agcgcgatca catggtcctg ctggagttcg tgaccgccgc cgggatcact 1800

ctcggcatgg acgagctgta caagtaa 1827

<210> 27
<211> 2814
<212> DNA
<213> Artificial Sequence

<220>
<223> PREY-CD105-EGFP plasmid

<400> 27
atggaccgcg gcacgctccc tctggctgtt gccctgctgc tggccagctg cagcctcagc 60

cccacaagtc ttgcaggcag tccccgcgag tacacctcct atatgcctca cggcaagccc 120

atccccaacc ccctgctggg cctggacagc accgaaacag tccattgtga ccttcagcct 180

gtgggccccg agaggggcga ggtgacatat accactagcc aggtctcgaa gggctgcgtg 240

gctcaggccc ccaatgccat ccttgaagtc catgtcctct tcctggagtt cccaacgggc 300

ccgtcacagc tggagctgac tctccaggca tccaagcaaa atggcacctg gccccgagag 360

gtgcttctgg tcctcagtgt aaacagcagt gtcttcctgc atctccaggc cctgggaatc 420

ccactgcact tggcctacaa ttccagcctg gtcaccttcc aagagccccc gggggtcaac 480

accacagagc tgccatcctt ccccaagacc cagatccttg agtgggcagc tgagaggggc 540

cccatcacct ctgctgctga gctgaatgac ccccagagca tcctcctccg actgggccaa 600

gcccaggggt cactgtcctt ctgcatgctg gaagccagcc aggacatggg ccgcacgctc 660

gagtggcggc cgcgtactcc agccttggtc cggggctgcc acttggaagg cgtggccggc 720

cacaaggagg cgcacatcct gagggtcctg ccgggccact cggccgggcc ccggacggtg 780

acggtgaagg tggaactgag ctgcgcaccc ggggatctcg atgccgtcct catcctgcag 840

ggtcccccct acgtgtcctg gctcatcgac gccaaccaca acatgcagat ctggaccact 900

ggagaatact ccttcaagat ctttccagag aaaaacattc gtggcttcaa gctcccagac 960

acacctcaag gcctcctggg ggaggcccgg atgctcaatg ccagcattgt ggcatccttc 1020

gtggagctac cgctggccag cattgtctca cttcatgcct ccagctgcgg tggtaggctg 1080

cagacctcac ccgcaccgat ccagaccact cctcccaagg acacttgtag cccggagctg 1140

ctcatgtcct tgatccagac aaagtgtgcc gacgacgcca tgaccctggt actaaagaaa 1200

gagcttgttg cgcatttgaa gtgcaccatc acgggcctga ccttctggga ccccagctgt 1260

gaggcagagg acaggggtga caagtttgtc ttgcgcagtg cttactccag ctgtggcatg 1320

caggtgtcag caagtatgat cagcaatgag gcggtggtca atatcctgtc gagctcatca 1380

ccacagcgga aaaaggtgca ctgcctcaac atggacagcc tctctttcca gctgggcctc 1440

tacctcagcc cacacttcct ccaggcctcc aacaccatcg agccggggca gcagagcttt 1500

gtgcaggtca gagtgtcccc atccgtctcc gagttcctgc tccagttaga cagctgccac 1560

ctggacttgg ggcctgaggg aggcaccgtg gaactcatcc agggccgggc ggccaagggc 1620

aactgtgtga gcctgctgtc cccaagcccc gagggtgacc cgcgcttcag cttcctcctc 1680

cacttctaca cagtacccat acccaaaacc ggcaccctca gctgcacggt agccctgcgt 1740

cccaagaccg ggtctcaaga ccaggaagtc cataggactg tcttcatgcg cttgaacatc 1800

atcagccctg acctgtctgg ttgcacaagc aaaggcctcg tcctgcccgc cgtgctgggc 1860

atcacctttg gtgccttcct catcggggcc ctgctcactg ctgcactctg gtacatctac 1920

tcgcacacgc gttcccccag caagcgggag cccgtggtgg cggtggctgc cccggcctcc 1980

tcggagagca gcagcaccaa ccacagcatc gggagcaccc agagcacccc ctgctccacc 2040

agcagcatgg caggaggagg tggaagcgga ggaggaggaa gcggaggagg aggtagcgtg 2100

agcaagggcg aggagctgtt caccggggtg gtgcccatcc tggtcgagct ggacggcgac 2160

gtaaacggcc acaagttcag cgtgtccggc gagggcgagg gcgatgccac ctacggcaag 2220

ctgaccctga agttcatctg caccaccggc aagctgcccg tgccctggcc caccctcgtg 2280

accaccctga cctacggcgt gcagtgcttc agccgctacc ccgaccacat gaagcagcac 2340

gacttcttca agtccgccat gcccgaaggc tacgtccagg agcgcaccat cttcttcaag 2400

gacgacggca actacaagac ccgcgccgag gtgaagttcg agggcgacac cctggtgaac 2460

cgcatcgagc tgaagggcat cgacttcaag gaggacggca acatcctggg gcacaagctg 2520

gagtacaact acaacagcca caacgtctat atcatggccg acaagcagaa gaacggcatc 2580

aaggtgaact tcaagatccg ccacaacatc gaggacggca gcgtgcagct cgccgaccac 2640

taccagcaga acacccccat cggcgacggc cccgtgctgc tgcccgacaa ccactacctg 2700

agcacccagt ccgccctgag caaagacccc aacgagaagc gcgatcacat ggtcctgctg 2760

gagttcgtga ccgccgccgg gatcactctc ggcatggacg agctgtacaa gtaa 2814

<210> 28
<211> 21
<212> DNA
<213> Artificial Sequence

<220>
<223> GAPDH_Forward

<400> 28
atgtgtccgt cgtggatctg a 21

<210> 29
<211> 20
<212> DNA
<213> Artificial Sequence

<220>
<223> GAPDH_Reverse

<400> 29
tgcctgcttc accaccttct 20

<210> 30
<211> 20
<212> DNA
<213> Artificial Sequence

<220>
<223> IL-10_Forward

<400> 30
actggcatga ggatcagcag 20

<210> 31
<211> 20
<212> DNA
<213> Artificial Sequence

<220>
<223> IL-10_Reverse

<400> 31
ctccttgatt tctgggccat 20

<210>    32
<211>    22
<212>    DNA
<213>    Artificial Sequence

<220>
<223>    IL-1b_Forward


<400>    32
gccacctttt gacagtgatg ag                                                22


<210>    33
<211>    20
<212>    DNA
<213>    Artificial Sequence

<220>
<223>    IL-1b_Reverse


<400>    33
atcaggacag cccaggtcaa                                                   20


<210>    34
<211>    20
<212>    DNA
<213>    Artificial Sequence

<220>
<223>    IL-13_Forward


<400>    34
atggcctctg taaccgcaag                                                   20


<210>    35
<211>    20
<212>    DNA
<213>    Artificial Sequence

<220>
<223>    IL-13_Reverse


<400>    35
tcctcattag aaggggccgt                                                   20


<210>    36
<211>    20
<212>    DNA
<213>    Artificial Sequence

<220>
<223>    TNF-a_Forward


<400>    36
cctgtagccc acgtcgtagc                                                   20

<210>	37
<211>	22
<212>	DNA
<213>	Artificial Sequence

<220>
<223>	TNF-a_Reverse


<400>	37
agcaatgact ccaaagtaga cc	22


<210>	38
<211>	20
<212>	DNA
<213>	Artificial Sequence

<220>
<223>	VCAM1_Forward


<400>	38
agttggggat tcggttgttc	20


<210>	39
<211>	20
<212>	DNA
<213>	Artificial Sequence

<220>
<223>	VCAM1_Reverse


<400>	39
cattccttac caccccattg	20


<210>	40
<211>	18
<212>	DNA
<213>	Artificial Sequence

<220>
<223>	E-selectin_Forward


<400>	40
ccagaatggc gtcatgga	18


<210>	41
<211>	20
<212>	DNA
<213>	Artificial Sequence

<220>
<223>	E-selectin_Reverse

<400>    41
taaagccctc attgcattga                                                    20

<210>    42
<211>    20
<212>    DNA
<213>    Artificial Sequence

<220>
<223>    ICAM1_Forward

<400>    42
caatttctca tgccgcacag                                                    20

<210>    43
<211>    21
<212>    DNA
<213>    Artificial Sequence

<220>
<223>    ICAM1_Reverse

<400>    43
agctggaaga tcgaaagtcc g                                                  21